(19) Europäisches Patentamt
European Patent Office
Office européen des brevets

(11) Veröffentlichungsnummer: 0 623 605 A2

(12) **EUROPÄISCHE PATENTANMELDUNG**

(21) Anmeldenummer: **94106319.0**

(22) Anmeldetag: **22.04.94**

(51) Int. Cl.[5]: **C07D 295/08**, C07D 295/14, C07D 311/58, A61K 31/495

(30) Priorität: **06.05.93 DE 4314962**

(43) Veröffentlichungstag der Anmeldung: **09.11.94 Patentblatt 94/45**

(84) Benannte Vertragsstaaten: **AT BE CH DE DK ES FR GB GR IE IT LI LU MC NL PT SE**

(71) Anmelder: **BAYER AG**

**D-51368 Leverkusen (DE)**

(72) Erfinder: **Wild, Hanno, dr.**
**Ausblick 128**
**D-42113 Wuppertal (DE)**
Erfinder: **Bender, Wolfgang, Dr.**
**Kaulbachstrasse 12**
**D-42113 Wuppertal (DE)**
Erfinder: **Häbich, Dieter, Dr.**
**Krummacherstrasse 82**
**D-42115 Wuppertal (DE)**
Erfinder: **Raddatz, Siegfried, Dr.**
**Jakob-Böhme-Strasse 21**
**D-51065 Köln (DE)**
Erfinder: **Röben, Wolfgang, Dr.**
**Strässchen Siefen 30**
**D-51467 Bergisch Gladbach (DE)**
Erfinder: **Seidel, Peter-Rudolf, Dr.**
**Alte Heide 5d**
**D-51147 Köln (DE)**
Erfinder: **Hansen, Jutta, Dr.**
**Pahlkestrasse 98**
**D-42115 Wuppertal (DE)**
Erfinder: **Paessens, Arnold, Dr.**
**Stresemannstrasse 51**
**D-42781 Haan (DE)**

(54) **Substituierte Piperazine als anti-retrovirale Mittel.**

(57) Die vorliegende Erfindung betrifft jetzt substituierte Piperazine der allgemeinen Formel (I)

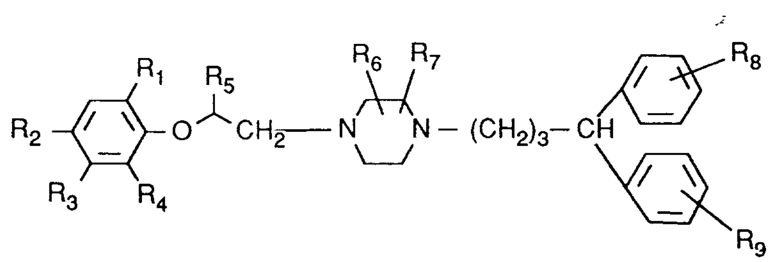

(I)

in welcher die Substituenten die in der Beschreibung angegebene Bedeutung haben, Verfahren zu ihrer Herstellung und ihre Verwendung als anti-retrovirale Mittel.

EP 0 623 605 A2

Die Erfindung betrifft substituierte Piperazine, Verfahren zu ihrer Herstellung und ihre Verwendung als anti-retrovirale Mittel.

Aus der Publikation WO 92/024 87-A (JP 3002-144 A) sind Ethylendiaminderivate mit einer ZNS-Wirkung bekannt. Außerdem werden Diarylalkyl-substituierte Alkylamine mit einer Herz-Kreislauf- und cerebrovaskulären Wirkung in der EP 0229 623 beschreiben. Die erfindungsgemäßen Verbindungen werden im Fall $R^5$ = H partiell vom Bedeutungsumfang dieser Publikation umfaßt.

Die vorliegende Erfindung betrifft jetzt substituierte Piperazine der allgemeinen Formel (I)

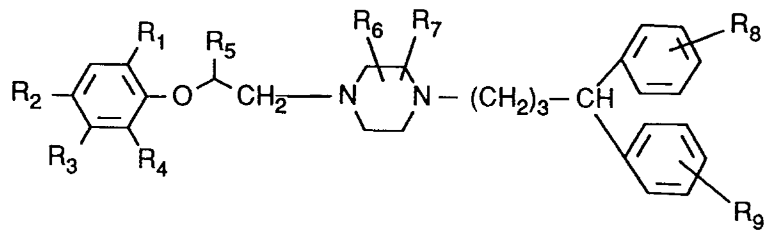

(I)

in welcher

| | |
|---|---|
| $R^1$, $R^2$, $R^3$ und $R^4$ | gleich oder verschieden sind und für Wasserstoff, Nitro, Halogen, Carboxy, Hydroxy, Formyl, geradkettiges oder verzweigtes Alkoxy oder Alkoxycarbonyl mit jeweils bis zu 8 Kohlenstoffatomen, Trifluormethyl, Phenyl oder für geradkettiges oder verzweigtes Alkyl mit bis zu 8 Kohlenstoffatomen stehen, das gegebenenfalls durch Hydroxy oder geschütztes Hydroxy substituiert ist, oder für eine Gruppe der Formel $-NR^{10}R^{11}$ oder -CO-A stehen, worin |
| $R^{10}$ und $R^{11}$ | gleich oder verschieden sind und Wasserstoff, Phenyl, Benzyl, eine Aminoschutzgruppe oder geradkettiges oder verzweigtes Alkyl mit bis zu 8 Kohlenstoffatomen bedeuten, oder |
| $R^{10}$ und $R^{11}$ | gemeinsam mit dem Stickstoffatom einen 5- bis 7-gliedrigen, gesättigten Heterocyclus bilden, |
| A | die oben angegebene Gruppe $-NR^{10}R^{11}$ oder den Rest der Formel |

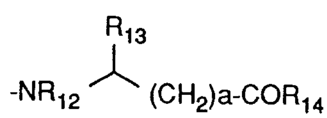

bedeutet,

| | |
|---|---|
| | worin |
| $R^{10}$ und $R^{11}$ | die oben angegebene Bedeutung haben, |
| $R^{12}$ | Wasserstoff oder geradkettiges oder verzweigtes Alkyl mit bis zu 4 Kohlenstoffatomen bedeutet, |
| $R^{13}$ | Cycloalkyl mit 3 bis 8 Kohlenstoffatomen oder Aryl mit 6 bis 10 Kohlenstoffatomen oder Wasserstoff bedeutet, oder geradkettiges oder verzweigtes Alkyl mit bis zu 8 Kohlenstoffatomen bedeutet, wobei das Alkyl gegebenenfalls durch Methylthio, Hydroxy, Mercapto, Guanidyl oder durch eine Gruppe der Formel $-NR^{15}R^{16}$ oder $R^{17}$-OC-substituiert ist, worin |
| $R^{15}$ und $R^{16}$ | unabhängig voneinander Wasserstoff, geradkettiges oder verzweigtes Alkyl mit bis zu 8 Kohlenstoffatomen oder Phenyl bedeuten, und |
| $R^{17}$ | Hydroxy, Benzyloxy, Alkoxy mit bis zu 6 Kohlenstoffatomen oder die oben aufgeführte Gruppe $-NR^{15}R^{16}$ bedeutet, worin |

| | |
|---|---|
| $R^{15}$ und $R^{16}$ | die oben angegebene Bedeutung haben, oder das Alkyl gegebenenfalls durch Cycloalkyl mit 3 bis 8 Kohlenstoffatomen oder durch Aryl mit 6 bis 10 Kohlenstoffatomen substituiert ist, das seinerseits durch Hydroxy, Halogen, Nitro, Alkoxy mit bis zu 8 Kohlenstoffatomen oder durch die Gruppe $-NR^{15}R^{16}$ substituiert ist, worin |
| $R^{15}$ und $R^{16}$ | die oben angegebene Bedeutung haben, oder das Alkyl gegebenenfalls durch einen 5- bis 6-gliedrigen stickstoffhaltigen Heterocyclus oder Indolyl substituiert ist, worin die entsprechenden -NH-Funktionen gegebenenfalls durch Alkyl mit bis zu 6 Kohlenstoffatomen oder durch eine Aminoschutzgruppe geschützt sind, und |
| a | die Zahl 0 oder 1 bedeutet, |
| $R^{14}$ | Hydroxy oder geradkettiges oder verzweigtes Alkoxy mit bis zu 8 Kohlenstoffatomen bedeutet, oder |
| $R^2$ und $R^3$ oder $R^3$ und $R^4$ | gemeinsam einen unter Einbezug der phenylischen Doppelbindung, partiell ungesättigten 5- bis 7-gliedrigen Carbocyclus bilden, |
| $R^5$ | für Wasserstoff oder für geradkettiges oder verzweigtes Alkyl mit bis zu 8 Kohlenstoffatomen steht, oder für eine Gruppe der Formel $-CO_2R^{18}$ steht, worin |
| $R^{18}$ | Wasserstoff, geradkettiges oder verzweigtes Alkyl mit bis zu 8 Kohlenstoffatomen oder eine Hydroxyschutzgruppe bedeutet, oder |
| $R^5$ | für eine Gruppe der Formel -CO-B steht, worin |
| B | die oben angegebene Bedeutung von A hat und mit dieser gleich oder verschieden ist, oder |
| $R^1$ und $R^5$ | unter Einbezug des Sauerstoffatoms und des Phenylrings zusammen einen 5- bis 6-gliedrigen, ungesättigten Heterocyclus bilden, |
| $R^6$ und $R^7$ | gleich oder verschieden sind und für Wasserstoff, Carboxy oder geradkettiges oder verzweigtes Alkyl mit bis zu 8 Kohlenstoffatomen stehen, das gegebenenfalls durch Hydroxy oder Phenyl substituiert ist, oder für geradkettiges oder verzweigtes Alkoxycarbonyl mit bis zu 8 Kohlenstoffatomen stehen, |
| $R^8$ und $R^9$ | gleich oder verschieden sind und für Halogen, Cyano, Nitro, Azido oder Hydroxy stehen, |

und deren Salze.

Physiologisch unbedenkliche Salze der substituierten Piperazine können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Als Salze können Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabietylamin, 1-Ephenamin oder Methylpiperidin.

Die erfindungsgemäßen Verbindungen können in stereoisomeren Formen existieren, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Aminoschutzgruppen im Rahmen der Erfindung sind die üblichen in der Peptid-Chemie verwendeten Aminoschutzgruppen. Hierzu gehören bevorzugt: Benzyloxycarbonyl, tert.Butoxycarbonyl oder Allyloxycarbonyl.

Hydroxyschutzgruppe steht im allgemeinen für Trimethylsilyl, Triethylsilyl, Triisopropylsilyl, tert.Butyldimethylsilyl, Triphenylsilyl, Trimethylsilylethoxycarbonyl, Benzyl, Benzyloxycarbonyl, tert.Butoxycarbonyl, Allyloxycarbonyl, Acetyl oder Tetrahydropyranyl. Bevorzugt sind Trimethylsilyl, Triethylsilyl, Triisopropylsilyl, tert.Butyl-dimethylsilyl, Triphenylsilyl, Benzyloxycarbonyl, tert.Butoxycarbonyl, Allyloxycarbonyl und Acetyl.

Bevorzugt sind Verbindungen der allgemeinen Formel (I),
in welcher

| | |
|---|---|
| $R^1$, $R^2$, $R^3$ und $R^4$ | gleich oder verschieden sind und für Wasserstoff, Nitro, Fluor, Chlor, Brom, Carboxy, Hydroxy, Formyl, geradkettiges oder verzweigtes Alkoxy oder Alkoxycarbonyl mit jeweils bis zu 6 Kohlenstoffatomen, Trifluormethyl, Phenyl oder<br>für geradkettiges oder verzweigtes Alkyl mit bis zu 6 Kohlenstoffatomen steht, das gegebenenfalls durch Hydroxy oder geschütztes Hydroxy substituiert ist, oder<br>für eine Gruppe der Formel $-NR^{10}R^{11}$ oder -CO-A stehen,<br>worin |
| $R^{10}$ und $R^{11}$ | gleich oder verschieden sind und Wasserstoff, Phenyl, Benzyloxycarbonyl, Butyl, tert.Butyloxycarbonyl (BOC), Acetyl, Benzyl oder geradkettiges oder verzweigtes Alkyl mit bis zu 6 Kohlenstoffatomen bedeuten,<br>oder |
| $R^{10}$ und $R^{11}$ | gemeinsam mit dem Stickstoffatom einen Pyrrolidin- oder Piperidinring bilden, |
| A | die oben angegebene Gruppe $-NR^{10}R^{11}$ oder den Rest der Formel |

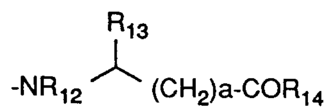 bedeutet,

| | |
|---|---|
| | worin |
| $R^{10}$ und $R^{11}$ | die oben angegebene Bedeutung haben, |
| $R^{12}$ | Wasserstoff, Methyl oder Ethyl bedeutet, |
| $R^{13}$ | Cyclopentyl, Cyclohexyl, Phenyl oder Wasserstoff bedeutet, oder<br>geradkettiges oder verzweigtes Alkyl mit bis zu 6 Kohlenstoffatomen bedeutet,<br>wobei das Alkyl gegebenenfalls durch Methylthio, Hydroxy, Mercapto, Guanidyl, Amino, Carboxy oder $H_2N$-CO-substituiert sein kann,<br>oder das Alkyl durch Cyclohexyl, Naphthyl oder Phenyl substituiert ist, das seinerseits durch Fluor, Hydroxy, Nitro oder Alkoxy mit bis zu 4 Kohlenstoffatomen substituiert sein kann,<br>oder das Alkyl durch Indolyl, Imidazolyl, Pyridyl, Triazolyl oder Pyrazolyl substituiert ist, wobei die entsprechenden -NH-Funktionen gegebenenfalls durch Alkyl mit bis zu 4 Kohlenstoffatomen oder durch eine Aminoschutzgruppe geschützt sind, |
| $R^{14}$ | Hydroxy oder geradkettiges oder verzweigtes Alkoxy mit bis zu 6 Kohlenstofatomen bedeutet, |
| a | die Zahl 0 oder 1 bedeutet,<br>oder |
| $R^2$ und $R^3$ oder $R^3$ und $R^4$ | jeweils gemeinsam, unter Einbezug der phenylischen Doppelbindung, einen Cyclohexenylring bilden, |
| $R^5$ | für Wasserstoff oder geradkettiges oder verzweigtes Alkyl mit bis zu 6 Kohlenstoffatomen steht, oder<br>für eine Gruppe der Formel $-CO_2R^{18}$ steht,<br>worin |

| | |
|---|---|
| $R^{18}$ | Wasserstoff, geradkettiges oder verzweigtes Alkyl mit bis zu 6 Kohlenstoffatomen, Acetyl oder Benzyl bedeutet, |
| | oder |
| $R^5$ | für eine Gruppe der Formel -CO-B steht, |
| | worin |
| B | die oben angegebene Bedeutung von A hat und mit dieser gleich oder verschieden ist, |
| | oder |
| $R^1$ und $R^5$ | unter Einbezug des Sauerstoffatoms und des Phenylrings zusammen einen Chromanring bilden, |
| $R^6$ und $R^7$ | gleich oder verschieden sind und für Wasserstoff, Carboxy oder geradkettiges oder verzweigtes Alkyl mit bis zu 6 Kohlenstoffatomen stehen, das gegebenenfalls durch Hydroxy oder Phenyl substituiert ist, oder für geradkettiges oder verzweigtes Alkoxycarbonyl mit bis zu 6 Kohlenstoffatomen stehen, |
| $R^8$ und $R^9$ | gleich oder verschieden sind und für Fluor, Chlor, Cyano, Nitro, Azido oder Hydroxy stehen, |

und deren Salze.

Besonders bevorzugt sind Verbindungen der allgemeinen Formel (I), in welcher

| | |
|---|---|
| $R^1$, $R^2$, $R^3$ und $R^4$ | gleich oder verschieden sind und für Wasserstoff, Nitro, Fluor, Chlor, Brom, Carboxy, Hydroxy, Formyl, geradkettiges oder verzweigtes Alkoxy oder Alkoxycarbonyl mit jeweils bis zu 4 Kohlenstoffatomen, Trifluormethyl, Phenyl oder für geradkettiges oder verzweigtes Alkyl mit bis zu 4 Kohlenstoffatomen stehen, das gegebenenfalls durch Hydroxy substituiert ist, oder für eine Gruppe der Formel $-NR^{10}R^{11}$ oder -CO-A stehen, |
| | worin |
| $R^{10}$ und $R^{11}$ | gleich oder verschieden sind und Wasserstoff, Phenyl, Benzyl, Benzyloxycarbonyl, Butyl, tert.Butyloxycarbonyl (BOC), Acetyl oder geradkettiges oder verzweigtes Alkyl mit bis zu 4 Kohlenstoffatomen bedeuten, |
| | oder |
| $R^{10}$ und $R^{11}$ | gemeinsam mit dem Stickstoffatom einen Pyrrolidin- oder Piperidinring bilden, |
| A | die oben angegebene Gruppe $-NR^{10}R^{11}$ oder den Rest der Formel 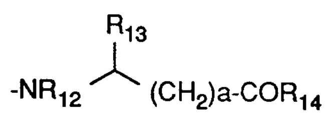 bedeutet, |
| | worin |
| $R^{10}$ und $R^{11}$ | die oben angegebene Bedeutung haben, |
| $R^{12}$ | Wasserstoff, Methyl oder Ethyl bedeutet, |
| $R^{13}$ | Cyclopentyl, Cyclohexyl, Phenyl oder Wasserstoff bedeutet, oder geradkettiges oder verzweigtes Alkyl mit bis zu 4 Kohlenstoffatomen bedeutet, wobei das Alkyl gegebenenfalls durch Methylthio, Hydroxy, Mercapto, Guanidyl, Amino, Carboxy oder $H_2N$-CO-substituiert sein kann, oder das Alkyl durch Cyclohexyl, Naphthyl oder Phenyl substituiert ist, das seinerseits durch Fluor, Hydroxy, Nitro oder Alkoxy mit bis zu 4 Kohlenstoffatomen substituiert sein kann, oder das Alkyl durch Indolyl, Imidazolyl, Pyridyl, Triazolyl oder Pyrazolyl substituiert ist, wobei die entsprechenden -NH-Funktionen gegebenenfalls durch Alkyl mit bis zu 4 Kohlenstoffatomen oder durch eine Aminoschutzgruppe geschützt sind, |
| $R^{14}$ | Hydroxy oder geradkettiges oder verzweigtes Alkoxy mit bis zu 4 Kohlen- |

| | |
|---|---|
| | stofatomen bedeutet, |
| a | die Zahl 0 oder 1 bedeutet, |
| | oder |
| $R^2$ und $R^3$ oder $R^3$ und $R^4$ | jeweils gemeinsam, unter Einbezug der phenylischen Doppelbindung, einen Cyclohexenylring bilden, |
| $R^5$ | für Wasserstoff oder geradkettiges oder verzweigtes Alkyl mit bis zu 4 Kohlenstoffatomen steht, oder |
| | für eine Gruppe der Formel $-CO_2R^{18}$ steht, |
| | worin |
| $R^{18}$ | Wasserstoff, geradkettiges oder verzweigtes Alkyl mit bis zu 4 Kohlenstoffaotmen, Acetyl oder Benzyl bedeutet, |
| | oder |
| $R^5$ | für eine Gruppe der Formel -CO-B steht, |
| | worin |
| B | die oben angegebene Bedeutung von A hat und mit dieser gleich oder verschieden ist, |
| | oder |
| $R^1$ und $R^5$ | unter Einbezug des Sauerstoffatoms und des Phenylrings zusammen einen Chromanring bilden, |
| $R^6$ und $R^7$ | gleich oder verschieden sind und für Wasserstoff, Carboxy oder geradkettiges oder verzweigtes Alkyl mit bis zu 4 Kohlenstoffatomen stehen, das gegebenenfalls durch Hydroxy oder Phenyl substituiert ist, oder für geradkettiges oder verzweigtes Alkoxycarbonyl mit bis zu 4 Kohlenstoffatomen stehen, |
| $R^8$ und $R^9$ | gleich oder verschieden sind und für Fluor, Chlor, Cyano, Nitro oder Hydroxy stehen, |

und deren Salze.

Außerdem wurden Verfahren zur Herstellung der erfindungsgemäßen Verbindungen der allgemeinen Formel (I) gefunden, dadurch gekennzeichnet, daß man

[A] Verbindungen der allgemeinen Formel (II)

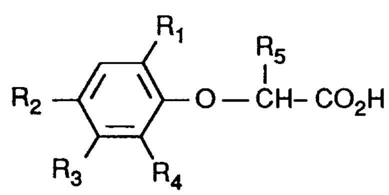

(II)

in welcher

$R^1$, $R^2$, $R^3$, $R^4$ und $R^5$ die oben angegebene Bedeutung haben,

zunächst durch Umsetzung mit Verbindungen der allgemeinen Formel (III)

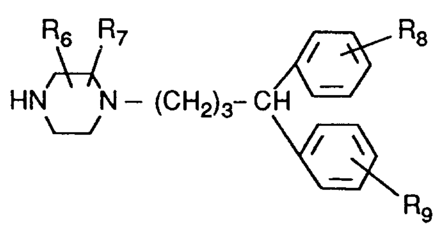

(III)

in welcher

$R^6$, $R^7$, $R^8$ und $R^9$ die oben angegebene Bedeutung haben,

in inerten Lösemitteln, in Anwesenheit einer Base und/oder Hilfsstoffes, gegebenenfalls unter vorgeschalteter Aktivierung der Carbonsäurefunktion,

in die Verbindungen der allgemeinen Formel (IV)

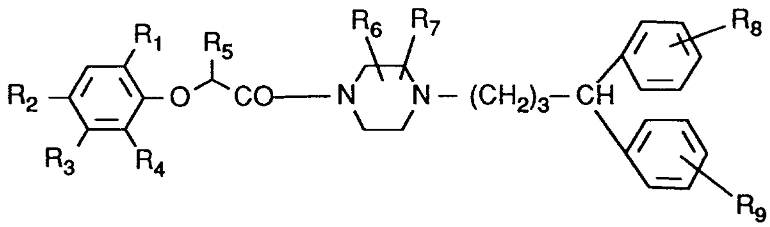

(IV)

in welcher

$R^1$, $R^2$, $R^3$, $R^4$, $R^5$, $R^6$, $R^7$, $R^8$ und $R^9$ die oben angegebene Bedeutung haben,

überführt und in einem zweiten Schritt nach üblichen Methoden die Carbonylfunktion reduziert,

oder

[B] Verbindungen der allgemeinen Formel (V)

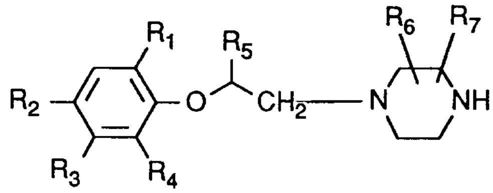

(V)

in welcher

$R^1$, $R^2$, $R^3$, $R^4$, $R^5$, $R^6$ und $R^7$ die oben angegebene Bedeutung haben,

zunächst durch Umsetzung mit Verbindungen der allgemeinen Formel (VI)

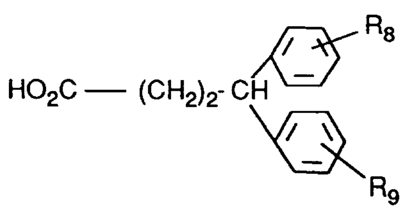

(VI)

in welcher

$R^8$ und $R^9$ die oben angegebene Bedeutung haben,

in inerten Lösemitteln, gegebenenfalls in Anwesenheit einer Base und/oder Hilfsstoffes, in die Verbindungen der allgemeinen Formel (VII)

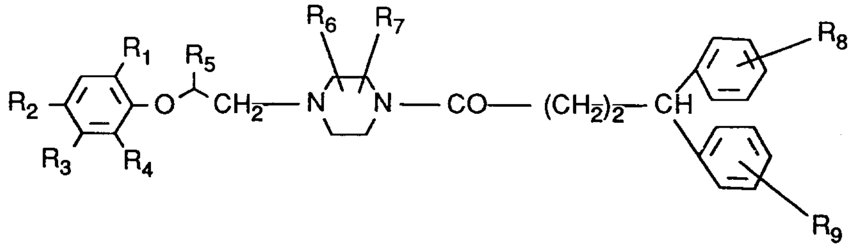

(VII)

in welcher

$R^1$, $R^2$, $R^3$, $R^4{}_7$ $R^5$, $R^6$, $R^7$, $R^8$ und $R^9$ die oben angegebene Bedeutung haben,

überführt, und anschließend die Carbonylfunktion reduziert,

oder

[C] Verbindungen der allgemeinen Formel (VIII)

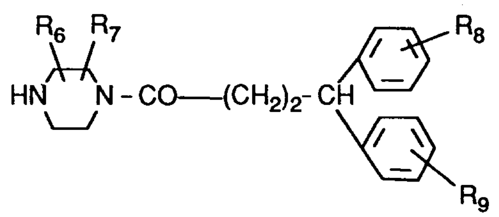

(VIII)

in welcher

$R^6$, $R^7$, $R^8$ und $R^9$ die oben angegebene Bedeutung haben,

zunächst durch Umsetzung mit Verbindungen der allgemeinen Formel (II), wie unter [A] beschrieben, in die Verbindungen der allgemeinen Formel (IX)

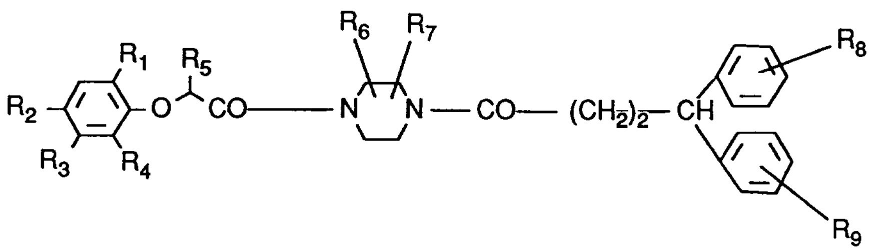

(IX)

in welcher

$R^1$, $R^2$, $R^3$, $R^4$, $R^5$, $R^6$, $R^7$, $R^8$ und $R^9$ die oben angegebene Bedeutung haben,

überführt und anschließend die Carbonylfunktion reduziert,

oder

[D] Verbindungen der allgemeinen Formel (X)

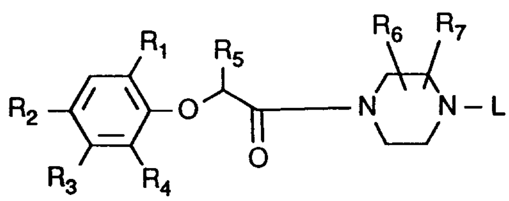

(X)

in welcher

$R^1$, $R^2$, $R^3$, $R^4$, $R^5$, $R^6$ und $R^7$ die oben angegebene Bedeutung haben

und

L für eine Aminoschutzgruppe, vorzugsweise Benzyl steht,

nach Abspaltung der Aminoschutzgruppe, zunächst mit Verbindungen der allgemeinen Formel (VI) in inerten Lösemitteln, gegebenenfalls in Anwesenheit einer Base und/oder Hilfsstoffes umsetzt und anschließend wie unter [A] beschrieben einer Reduktion der Carbonylfunktion durchführt,

und im Fall der freien Carbonsäuren, den jeweiligen Ester mit Säuren oder Basen hydrolysiert oder verseift,

und im Fall einer Aminosäuregruppierung, diese nach denen in der Peptidchemie üblichen Methoden, beispielsweise durch Aktivierung der Carbonsäurefunktion, Blockierung und Deblockierung der entsprechenden Carbonsäure und/oder Aminosäurefunktionen und Kondensation, herstellt,

und im Fall der Substituenten $R^1$, $R^2$, $R^3$, $R^4$, $R^5$, $R^6$, $R^7$, $R^8$ und $R^9$, diese nach bekannten Verfahren, beispielsweise durch Reduktion, Alkylierung, Verseifung variiert.

Die erfindungsgemäßen Verfahren können durch folgendes Formelschema beispielhaft erläutert werden:

[A]

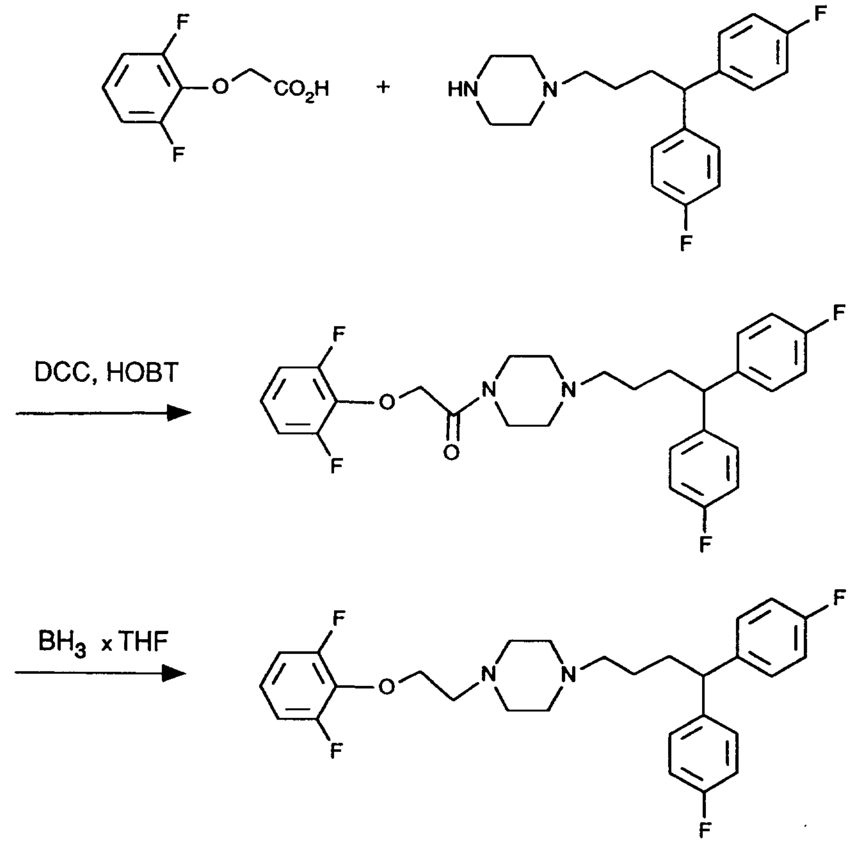

[B]

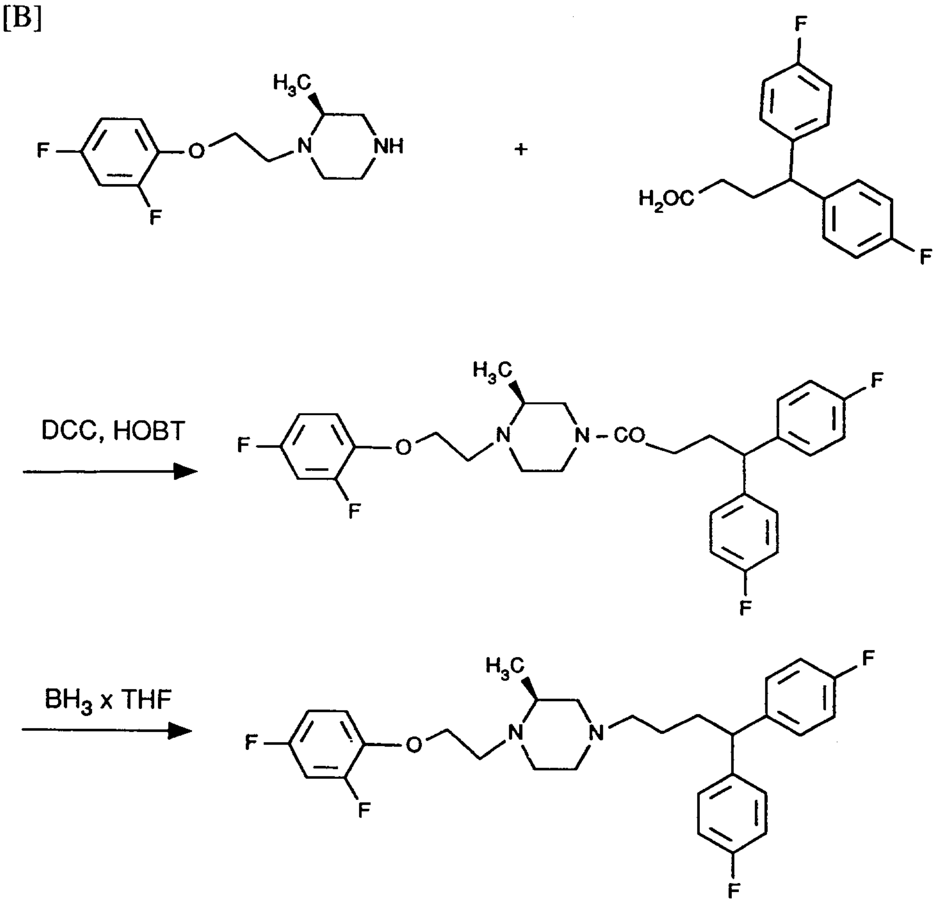

[C]

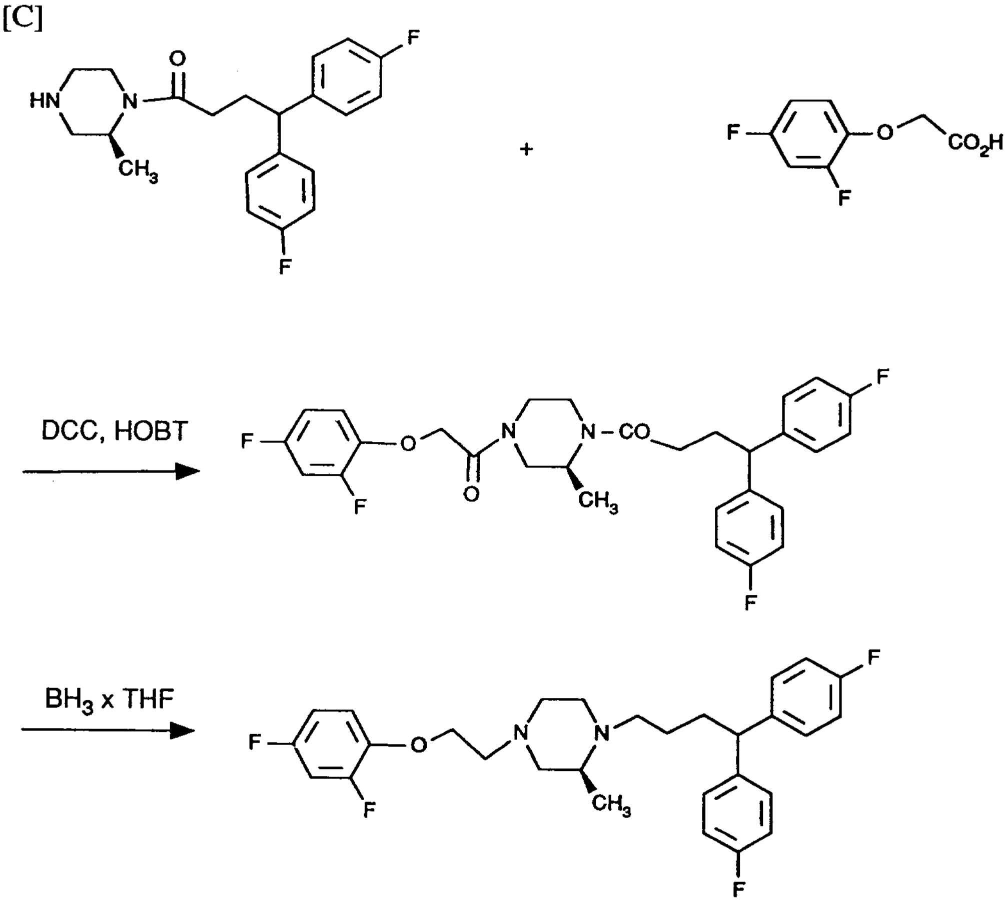

[D]

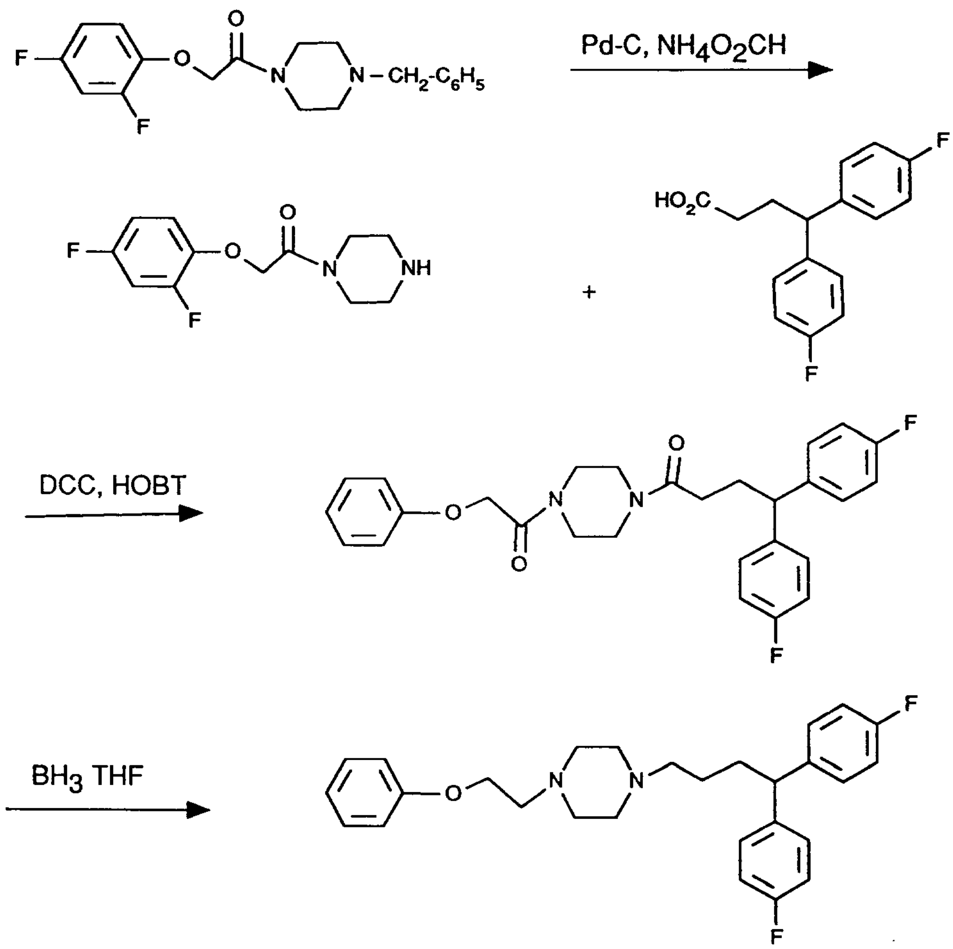


Als Lösemittel eignen sich die üblichen inerten Lösemittel, die sich unter den Reaktionsbedingungen nicht verändern. Hierzu gehören bevorzugt organische Lösemittel wie Ether z.B. Diethylether, Glykolmono- oder -dimethylether, Dioxan oder Tetrahydrofuran, oder Kohlenwasserstoffe wie Benzol, Toluol, Xylol, Cyclohexan oder Erdölfraktionen oder Halogenkohlenwasserstoffe wie Methylenchlorid, Chloroform, Tetrachlorkohlenstoff, oder Dimethylsulfoxid, Dimethylformamid, Hexamethylphosphorsäuretriamid, Essigester, Pyridin, Triethylamin oder Picolin. Ebenso ist es möglich, Gemische der genannten Lösemittel zu verwenden. Besonders bevorzugt sind Dichlormethan, Chloroform, DImethylformamid oder Tetrahydrofuran.

Als Basen für die erfindungsgemäßen Verfahren können im allgemeinen anorganische oder organische Basen eingesetzt werden. Hierzu gehören vorzugsweise Alkalihydroxide wie zum Beispiel Lithiumhydroxid, Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate, Calcium- oder Caesiumcarbonat, oder Alkali- oder Erdalkalialkoholate oder -amide wie Natrium- oder Kaliummethanolat, Natrium- oder Kaliumethanolat oder Kalium-tert.butylat, oder Lithiumdiisopropylamid (LDA), oder organische Amine (Trialkyl($C_1$-$C_6$)-amine) wie Triethylamin, oder Heterocyclen wie 1,4-Diazabicyclo[2.2.2]octan (DABCO), 1,8-Diazabicyclo[5.4.0]-undec-7-en (DBU), Pyridin, Diaminopyridin, Methylpiperidin oder Morpholin. Es ist auch möglich als Basen Alkalimetalle, wie Natrium oder deren Hydride wie Natriumhydrid einzusetzen. Bevorzugt ist Kaliumcarbonat, Natriumhydrid, Kalium-tert.-butylat oder Caesiumcarbonat.

Im allgemeinen setzt man die Base in einer Menge von 0,05 mol bis 10 mol, bevorzugt von 1 mol bis 2 mol, jeweils bezogen auf 1 mol der Verbindungen der Formeln (III), (V) und (VIII) ein.

Die erfindungsgemäßen Verfahren werden im allgemeinen in einem Temperaturbereich von -100 °C bis +100 °C, bevorzugt von 0 °C bis 80 °C, durchgeführt.

Die erfindungsgemäßen Verfahren werden im allgemeinen bei Normaldruck durchgeführt. Es ist aber auch möglich, die Verfahren bei Überdruck oder bei Unterdruck durchzuführen (z.B. in einem Bereich von

0,5 bis 5 bar).

Als Hilfsstoffe werden bevorzugt Kondensationsmittel eingesetzt, die auch Basen sein können, insbesondere wenn die Carboxylgruppe als Anhydrid aktiviert vorliegt. Bevorzugt werden hier die üblichen Kondensationsmittel wie Carbodiimide z.B. N,N'-Diethyl-, N,N'-Diisopropyl-, N,N'-Dicyclohexylcarbodiimid, N-(3-Dimethylaminoisopropyl)-N'-ethylcarbodiimid-Hydrochlorid, N-Cyclohexyl-N'-(2-morpholinoethyl)-carbodiimid-metho-p-toluolsulfonat, oder Carbonylverbindungen wie Carbonyldiimidazol, oder 1,2-Oxazoliumverbindungen wie 2-Ethyl-5-phenyl-1,2-oxazolium-3-sulfat oder 2-tert-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Benzotriazolyloxytris(dimethylamino)phosphonium-hexafluorophosphat oder 1-Hydroxybenzotriazol.

Außerdem können beispielsweise Alkalicarbonate, z.B. Natrium- oder Kaliumcarbonat oder -hydrogencarbonat, oder organische Basen wie Trialkylamine, z.B. Triethylamin, Ethyldiisopropylamin, N-Ethylmorpholin, N-Methylpiperidin oder N-Methylmorpholin eingesetzt werden. Bevorzugt ist N-Methylmorpholin.

Die Hilfsstoffe werden in einer Menge von 1,0 Mol bis 3,0 Mol, bevorzugt 1,0 bis 1,2 Mol, bezogen auf jeweils 1 Mol der Verbindungen der allgemeinen Formeln (III), (VI) und (VIII) eingesetzt.

Die Reaktionen werden in einem Temperaturbereich von -30 °C bis 100 °C, vorzugsweise bei 0 °C bis 30 °C und bei Normaldruck, durchgeführt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Hydrolyse wird im allgemeinen in einem Temperaturbereich von 0 °C bis +100 °C, bevorzugt von +20 °C bis +80 °C, durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 Mol, bevorzugt von 1 bis 1,5 Mol, bezogen auf 1 Mol des Esters, eingesetzt. Besonders bevorzugt verwendet man molare Mengen der Reaktanden.

Die Hydrolyse von tert.-Butylestern erfolgt im allgemeinen mit Säuren, wie beispielsweise Salzsäure oder Trifluoressigsäure, in Anwesenheit eines der oben angegebenen Lösemitteln und/oder Wasser oder deren Gemische, vorzugsweise mit Dioxan, Tetrahydrofuran oder Dichlormethan.

Als Lösemittel für die Peptidknüpfung eignen sich die üblichen organischen Lösemittel, die sich unter den Reaktionsbedingungen nicht verändern. Hierzu gehören bevorzugt organische Lösemittel wie Alkohole, z.B. Methanol, Ethanol oder n-Propanol, Ether, z.B. Diethylether, Glykolmono- oder -dimethylether, Dioxan oder Tetrahydrofuran, oder Kohlenwasserstoffe wie Benzol, Toluol, Xylol, Cyclohexan oder Erdölfraktionen oder Halogenkohlenwasserstoffe wie Methylenchlorid, Dichlorethan (DCE), Chloroform, Tetrachlorkohlenstoff, oder Dimethylsulfoxid, Dimethylformamid, Hexamethylphosphorsäuretriamid, Essigester, Pyridin, Triethylamin oder Picolin. Ebenso ist es möglich, Gemische der genannten Lösemittel zu verwenden. Besonders bevorzugt sind Dichlormethan, Dichlorethan, Dimethylformamid oder n-Propanol.

Als Hilfsstoffe für die jeweiligen Peptidkupplungen werden bevorzugt Kondensationsmittel eingesetzt, die auch Basen sein können, insbesondere wenn die Carboxylgruppe als Anhydrid aktiviert vorliegt. Bevorzugt werden hier die üblichen Kondensationsmittel wie Carbodiimide z.B. N,N'-Diethyl-, N,N'-Dipropyl-, n,n'-Diisopropyl-, N,N'-Dicyclohexylcarbodiimid, N-(3-Dimethylaminoisopropyl)-N'-ethylcarbodiimid-Hydrochlorid, oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Benzotriazolyloxy-tri-(dimethylamino)phosphoniumhexafluorophosphat, oder 1-Hydroxybenzotriazol und als Basen Alkalicarbonate, z.B. Natrium- oder Kaliumcarbonat, oder -hydrogencarbonat, oder organische Basen wie Trialkylamine z.B. Triethylamin, N-Ethylmorpholin, N-Methylpiperidin oder Diisopropylethylamin eingesetzt. Besonders bevorzugt sind Dicyclohexylcarbodiimid, N-Methylmorpholin und 1-Hydroxybenztriazol.

Die Umsetzungen werden im allgemeinen in einem Temperaturbereich von -20 °C bis +80 °C, bevorzugt von 0 °C bis +60 °C durchgeführt.

Im allgemeinen wird die Umsetzung bei Normaldruck durchgeführt. Es ist aber auch möglich, bei Unterdruck oder bei Überdruck zu arbeiten (z.B. von 0,5 bis 5 bar).

Die Abspaltung der Aminoschutzgruppen kann ebenfalls nach üblicher Methode mit Säuren, wie beispielsweise Chlorwasserstoffsäure oder Trifluoressigsäure erfolgen.

Die Abspaltung der Aminoschutzgruppen erfolgt in an sich bekannter Weise unter sauren oder basischen Bedingungen, oder reduktiv durch katalytische Hydrierung beispielsweise mit Pd/C in organischen Lösemitteln wie Ethern, z.B. Tetrahydrofuran oder Dioxan, oder Alkoholen z.B. Methanol, Ethanol oder Isopropanol.

Die Hydrierung erfolgt im allgemeinen in einem Temperaturbereich von 0 °C bis 80 °C, vorzugsweise von 0 °C bis 40 °C.

Im allgemeinen wird die Hydrierung bei einem erhöhten Druck von 2 bar bis 8 bar, vorzugsweise von 3 bis 5 bar, durchgeführt.

Ebenso ist es möglich, insbesondere im Fall von Acylaminoschutzgruppen, diese in einem der oben angegebenen Ethern, mit Hydriden, wie beispielsweise Lithiumaluminiumhydrid oder Natriumborhydrid, bei Raumtemperatur und Normaldruck abzuspalten.

Die Reduktion von Nitroverbindungen ($R^1$, $R^2$, $R^3$ und/oder $R^4$) zu den entsprechenden Aminoverbindungen erfolgt nach üblichen Methoden, vorzugsweise aber im System Ammoniumchlorid/Fe/$H_2O$ in 2-Methoxyethanol in einem Temperaturbereich von +10 °C bis +150 °C, vorzugsweise von +80 °C bis +130 °C und Normaldruck.

Die Alkylierung von Aminogruppen erfolgt entweder mit Sulfonsäureestern oder substituierten oder unsubstituierten ($C_1$-$C_8$)-Dialkyl- oder ($C_1$-$C_8$)-Diarylsulfonate, vorzugsweise Methyliodid oder Dimethylsulfat, oder mit Formaldehyd/Natriumborhydrid in einem der oben aufgeführten Ether, vorzugsweise Tetrahydrofuran, in Anwesenheit von Säuren.

Die Alkylierung erfolgt im allgemeinen in einem der oben aufgeführten Lösemitteln, vorzugsweise in Dimethylformamid in einem Temperaturbereich von 0 °C bis +70 °C, vorzugsweise von 0 °C bis +30 °C und Normaldruck.

Die Reduktionen der Carbonylfunkionen der Verbindungen der allgemeinen Formeln (IV) und (VII) sowie partiell auftretender Zwischenprodukte mit dieser Gruppierung erfolgten im allgemeinen mit Reduktionsmitteln, wie beispielsweise Lithiumaluminiumhydrid, mit Boranlösung in Tetrahydrofuran oder mit Boran-Dimethylsulfid (Komplex in Tetrahydrofuran, in einem Temperaturbereich von 0 °C bis +70 °C, bevorzugt von +20 °C bis +65 °C und Normaldruck und anschließender Aufnahme in Säuren. Bevorzugt ist Boran-Lösung in Tetrahydrofuran.

Als Säuren für einzelne Verfahrensschritte eignen sich im allgemeinen Protonensäuren wie beispielsweise Salzsäure oder Schwefelsäure. Bevorzugt wird Schwefelsäure eingesetzt.

Die Säure wird im allgemeinen in einer Menge von 1 mol bis 20 mol, bevorzugt von 1 mol bis 5 mol, jeweils bezogen auf 1 mol des Reaktanden, eingesetzt.

Die Verbindungen der allgemeinen Formel (II) sind teilweise bekannt oder neu und können dann beispielsweise hergestellt werden, indem man Verbindungen der allgemeinen Formel (XI)

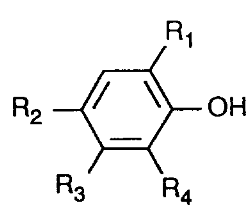

(XI)

in welcher

$R^1$, $R^2$, $R^3$ und $R^4$ die oben angegebene Bedeutung haben,

zunächst mit Verbindungen der allgemeinen Formel (XII)

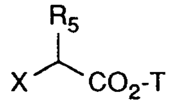

(XII)

in welcher

$R^5$ die oben angegebene Bedeutung hat,

X für Halogen, vorzugsweise für Brom steht

und

T für $C_1$-$C_4$-Alkyl steht,
in einem der oben aufgeführten Lösemittel und einer Base, vorzugsweise Aceton und Kaliumcarbonat, umsetzt und anschließend, wie oben beschrieben, den Ester verseift.

Die Reaktion erfolgt im allgemeinen in einem Temperaturbereich von +20°C bis +100°C, vorzugsweise von +20°C bis +70°C und Normaldruck.

Die Verbindungen der allgemeinen Formeln (XI) und (XII) sind an sich bekannt.

Die Verbindungen der allgemeinen Formel (III) sind an sich bekannt.

Die Verbindungen der allgemeinen Formel (IV) sind neu und können beispielsweise, wie oben beschrieben, hergestellt werden.

Die Verbindungen der allgemeinen Formel (V) sind teilweise bekannt oder neu und können dann beispielsweise hergestellt werden, indem man Verbindungen der allgemeinen Formel (II) zunächst mit Verbindungen der allgemeinen Formel (XIII)

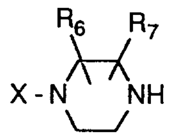 (XIII)

in welcher
$R^6$, $R^7$ und X die oben angegebene Bedeutung haben,
wie oben unter Verfahren [A] beschrieben umsetzt und anschließend die Carbonylfunktion, ebenfalls wie oben beschrieben, reduziert.

Die Verbindungen der allgemeinen Formel (XIII) sind bekannt.

Die Verbindungen der allgemeinen Formel (VI) sind bekannt.

Die Verbindungen der allgemeinen Formel (VII) sind größtenteils neu und können, wie oben beschrieben, hergestellt werden.

Die Verbindungen der allgemeinen Formel (VIII) sind teilweise bekannt oder neu und können dann beispielsweise hergestellt werden, indem man Verbindungen der allgemeinen Formel (XIII) mit 4,4-Bis-(substituierten 4-phenyl)butansäuren in einem der oben aufgeführten substituierten Lösemittel, Basen und Hilfsstoffen, vorzugsweise im System Tetrahydrofuran / Dicyclohexylcarbodiimid / Hydroxybenzotriazol in einem Temperaturbereich von 0°C bis +30°C, bevorzugt bei Raumtemperatur und Normaldruck umsetzt und anschließend mit Ammoniumformiat/Pd/C in Ethanol/Wasser die Benzylgruppe in einem Temperaturbereich von +20°C bis +100°C, bevorzugt von +30°C bis +50°C abspaltet.

Die Verbindungen der allgemeinen Formel (X) sind größtenteils neu und können beispielsweise hergestellt werden, indem man Verbindungen der allgemeinen Formel (II) mit Verbindungen der allgemeinen Formel (XIII), ebenfalls im System Hydroxybenzotriazol/Dicyclohexylcarbodiimid/Tetrahydrofuran bei Raumtemperatur und Normaldruck umsetzt.

Die hier beschriebenen Inhibitoren sind Inhibitoren der HIV-Protease und sind als solche für alle Zwecke einsetzbar, für die Enzyminhibitoren geeignet sind. Dies ist zum Beispiel der Einsatz in der Diagnostik, um die Präzision und Selektivität von Enzymaktivitätsmessungen zu verbessern. Bei der Affinitätschromatographie können sie als Affinitätslabel dienen und in der Forschung kann man sie zur Aufklärung von Reaktionsmechanismen und der Spezifität enzymatischer Reaktionen verwenden.

Darüber hinaus wurde überraschend gefunden, daß die Verbindungen der allgemeinen Formel (I) eine außerordentlich starke Wirkung gegen Retroviren besitzen. Dies wird mit einem HIV-spezifischen Protease-Enzymtest belegt.

Die Ergebnisse der unten aufgeführten Beispiele wurden nach dem in den folgenden Literaturangaben [vgl. Hansen, J., Billich, S., Schulze, T., Sukrow, S. and Mölling, K. (1988), EMBO Journal, Vol, 7, No. 6, pp. 1785 - 1791] beschriebenen HIV-Testsystem ermittelt: Gereinigte HIV-Protease wurde mit synthetischem Peptid, das eine Schnittstelle im Gag-Precursor-Protein imitiert und eine in vivo-Spaltstelle der HIV-Protease darstellt, inkubiert Die entstandenen Spaltprodukte des synthetischen Peptids wurden über Reverse Phase High Performance Liquid Chromatography (RP-HPLC) analysiert. Die angegebenen $IC_{50}$-Werte beziehen sich auf die Substanzkonzentration, die unter den oben aufgeführten Testbedingungen eine 50%ige Hemmung der Protease-Aktivität bewirkt.

Tabelle 1

| Bsp.-Nr. | % Inhibition bei 240 µg/ml | $IC_{50}$(µM) |
|---|---|---|
| **3** | **76** | **-** |
| **10** | **77** | **4,8** |
| **13** | **91** | **0,48** |
| **14** | **99** | **1,1** |
| **15** | **92** | **0,4** |
| **16** | **59** | **-** |
| **17** | **86** | **0,5** |
| **22** | **81** | **-** |
| **26** | **97** | **1,0** |
| **27** | **78** | **-** |
| **30** | **81** | **-** |
| **32** | **67** | **-** |
| **33** | **56** | **-** |
| **34** | **99** | **0,17** |
| **37** | **61** | **-** |
| **39** | **77** | **1,0** |
| **40** | **84** | **-** |
| **41** | **95** | **2,1** |
| **44** | **99** | **26** |

Die erfindungsgemäßen Verbindungen stellen wertvolle Wirkstoffe zur Behandlung und Prophylaxe von Erkrankungen, hervorgerufen durch Retroviren, in der Human- und Tiermedizin dar.

Als Indikationsgebiete in der Humanmedizin können beispielsweise genannt werden:

1.) Die Behandlung und Prophylaxe von menschlichen Retrovirusinfektionen.

2.) Für die Behandlung oder Prophylaxe von durch HIV I (Virus der humanen Immundefizienz; früher HTLV III/LAV genannt) und HIV II verursachten Erkrankungen (AIDS) und den damit assoziierten Stadien wie ARC (AIDS related complex) und LAS (Lymphadenopathie-Syndrom) sowie der durch dieses Virus verursachten Immunschwäche und Encephalopathie.

3.) Für die Behandlung oder die Prophylaxe einer HTLV-I oder HTLV-II Infektion.

4.) Für die Behandlung oder die Prophylaxe des AIDS-carrier Zustandes (AIDS-Überträger-Zustand).

Als Indikationen in der Tiermedizin können beispielsweise angeführt werden:

Infektionen mit

a) Maedi-visna(bei Schafen und Ziegen)

b) progressivem Pneumonievirus(PPV)(bei Schafen und Ziegen)

c) caprine arthritis encephalitis Virus(bei Schafen und Ziegen)

d) Zwoegersiekte Virus(bei Schafen)

e) infektiösem Virus der Anämie(des Pferdes)

f) Infektionen verursacht durch das Katzenleukämievirus

g) Infektionen verursacht durch das Virus der Katzen-Immundefizienz(FIV)

h) Infektionen verursacht durch das Virus der Affen-Immundefizienz(SIV)

Bevorzugt werden aus dem Indikationsgebiet in der Humanmedizin die oben aufgeführten Punkte 2, 3 und 4.

Zur vorliegenden Erfindung gehören pharmazeutische Zubereitungen, die neben nicht-toxischen, inerten pharmazeutisch geeigneten Trägerstoffen eine oder mehrere Verbindungen der Formel (I) enthalten oder die aus einem oder mehreren Wirkstoffen der Formel (I) bestehen, sowie Verfahren zur Herstellung dieser Zubereitungen.

Die Wirkstoffe der Formel (I) sollen in den oben aufgeführten pharmazeutischen Zubereitungen, in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den Verbindungen der Formel (I) auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 1 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 80, insbesondere 1 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

## Ausgangsverbindungen

### Beispiel I

(2,6-Difluorphenoxy)essigsäure

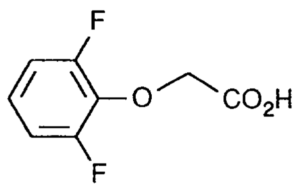

4 g (30.8 mmol) 2,6-Difluorphenol, 4,3 g (30,8 mmol) Kaliumcarbonat und 4,7 ml (42,3 mmol) Bromessigsäureethylester werden in 50 ml Aceton 18 h unter Rückfluß erhitzt. Dann wird vom Feststoff abgesaugt, das Filtrat mit 200 ml Ether verdünnt und mit verdünnter Salzsäure dreimal gewaschen. Die organische Phase wird anschließend getrocknet ($MgSO_4$) und in vacuo eingeengt. Man erhält 6,46 g eines Rohproduktes, das in 800 ml Ethanol gelöst und mit 120 ml 1 N Natronlauge versetzt wird. Man rührt 1 h bei RT, engt dann in vacuo auf 100 ml ein, säuert mit 6 N HCl auf pH 1 an und extrahiert 5 mal mit Dichlormethan. Die organischen Phasen werden getrocknet ($MgSO_4$) und eingeengt. Zur Reinigung wird der Rückstand in ges. $NaHCO_3$-Lösung aufgenommen und zweimal mit Ether gewaschen. Die wäßrige Phase wird dann erneut angesäuert und wie oben beschrieben weiter bearbeitet. Nach Einengen erhält man 4,4g (93%) der Carbonsäure.

### Beispiel II

1-[4,4-Bis-(4-fluorphenyl)butyl]-4-[(2,6-difluorphenoxy)acetyl]piperazin

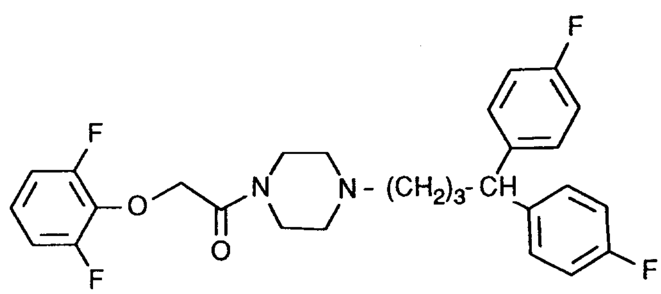

2,5 g (13,3 mmol) der Säure aus Beispiel I, 1,81 g (13,3 mmol) Hydroxybenzotriazol, 3,03 g (14,7 mmol) Dicyclohexylcarbodiimid und 4,4 g (13,3 mmol) 1-[4,4-Bis-(4-fluorphenyl)-butyl]piperazin werden in 100 ml Tetrahydrofuran 36 h bei RT gerührt. Dann wird vom Feststoff abgesaugt, das Filtrat eingeengt, der Rückstand in Essigester aufgenommen und 3 mal mit Wasser gewaschen. Die organische Phase wird dann getrocknet ($MgSO_4$) und eingeengt, und der Rückstand an Kieselgel mit Dichlormethan/Methanol (50:1) chromatographiert.

Ausbeute: 4,58 g (68,5%)
$R_f(CH_2Cl_2/CH_3OH\ 50:1) = 0,27$

**Beispiel III**

2,4-Difluorphenoxymalonsäurediethylester

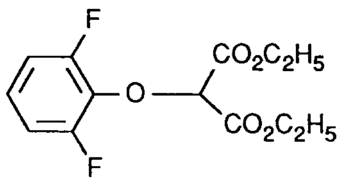

Zu einer Lösung von 38,1 g (0,54 mol) Natriumethanolat in 750 ml Ethanol werden unter Eiskühlung 63,6 g (0,49 mol) 2,4-Difluorphenol zugegeben. Nach 1 h bei RT werden 99,6 g (0,51 mol) Chlormalonsäurediethylester in 250 ml Ethanol zugefügt und 18 h bei RT gerührt. Dann wird mit 1 N HCl pH 7 eingestellt, filtriert und das Filtrat eingeengt. Der Rückstand wird mit Dichlormethan an Kieselgel chromatographiert.
Ausbeute: 47 g (34%)

**Beispiel IV**

(R/S)-2,4-Difluorphenoxymalonsäuremonoethylester

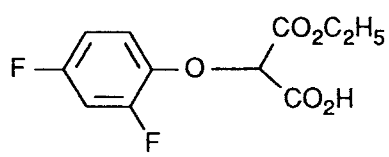

39,5 g (0,14 mol) des Diesters aus Beispiel III in 700 ml THF werden bei 0 °C mit 150 ml 1 N NaOH versetzt. Nach 3 h bei 0 °C wird mit 1 N HCl pH 2 eingestellt und bis auf ca. 100 ml eingeengt. Man extrahiert 3 mal mit Essigester, trocknet ($MgSO_4$) und engt ein. Der Rückstand wird an Kieselgel mit Dichlormethan/Ethanol (5:2) chromatographiert.
Ausbeute: 17,1 g (48%)

**Beispiel V**

(S)-1-[4,4-Bis-(4-fluorphenyl)butanoyl]-2-methyl-piperazin

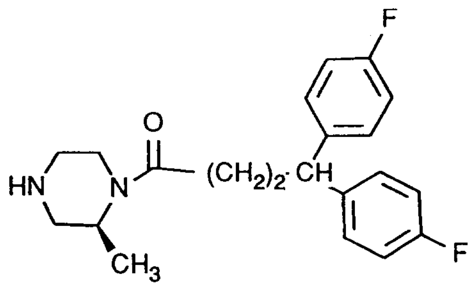

(S)-1-Benzyl-3-methylpiperazin und 4,4-Bis-(4-fluorphenyl)butansäure werden wie unter Beispiel II beschrieben zum Amid umgesetzt. 556 mg (1,24 mmol) dieser Verbindung werden in 2,9 ml Ethanol und 0,6 ml Wasser mit 195 mg (3,1 mmol) Ammoniumformiat und 195 mg Palladium auf Aktivkohle (10%) bei 50 °C 30 min gerührt. Dann wird filtriert, das Filtrat eingeengt und der Rückstand an Kieselgel mit Dichlormethan/Methanol (9:1) als Eluens chromatographiert.
Ausbeute: 389 mg (88%)
$R_f$ (Dichlormethan/Methanol 9:1) = 0,27

**Herstellungsbeispiele**

**Beispiel 1**

1-[4,4-Bis-(4-fluorphenyl)butyl]-4-[2-(2,6-difluorphenoxy)ethyl]piperazin

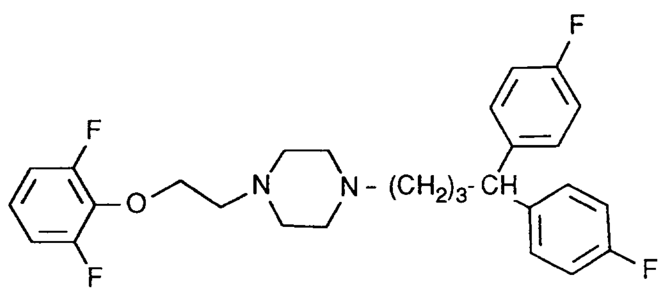

4,06 g (8,1 mmol) des Amids aus Beispiel II werden in 100 ml abs. Tetrahydrofuran (THF) mit 32,6 ml (32,6 mmol) einer 1 M Boran-Lösung in THF versetzt. Man läßt über Nacht bei RT stehen, trägt dann in 100 ml 5 N HCl ein und erhitzt 20 min auf 60 °C. Nach dem Abkühlen wird mit konz. Natronlauge pH 11 eingestellt und 3 mal mit Essigester extrahiert. Die Vereinigten organischen Phasen werden getrocknet ($MgSO_4$) und eingeengt und der Rückstand an Kieselgel mit Essigester/Aceton (20:1) als Eluens chromtographiert.
Ausbeute: 3,7 g, Öl
$R_f$(Essigester/Aceton 10:1) = 0,52
Das ölige Produkt wird in 50 ml Ether gelöst und mit 10 ml ges. etherischer Salzsäure versetzt. Der erhaltene Feststoff (das Dihydrochlorid der Titelverbindung) wird abgesaugt, mit Ether gewaschen und getrocknet.
Ausbeute: 3,5 g

## Beispiel 2

1-[4,4-Bis(4-fluorphenyl)butyl]-4-[2-(2-aminophenoxy)ethyl]piperazin

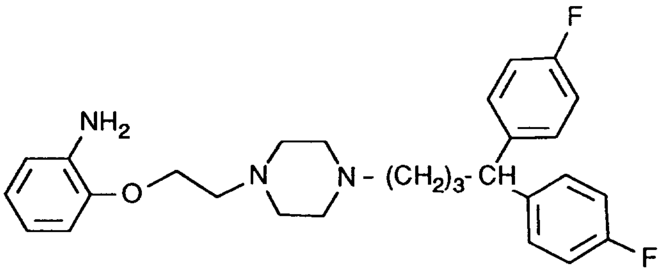

2-Nitrophenol wird, wie für Beispiel 1 beschrieben, in 1-[4,4-Bis-(4-fluorphenyl)butyl]-4-[2-(2-nitrophenoxy)-ethyl]piperazin überführt. 1 g (2,02 mmol) dieser Substanz (freie Base) in 8 ml 2-Methoxyethanol wird mit 646 mg (12,1 mmol) Ammoniumchlorid und 2 ml Wasser auf Rückfluß erhitzt und portionsweise mit 646 mg Eisen-Spänen versetzt (ca. 15 min.). Man erhitzt 3 h unter Rückfluß, kühlt ab, saugt über Kieselgur ab, wäscht mit heißem Methanol nach und engt das Filtrat in vacuo ein. Der Rückstand wird mit Dichlormethan/Methanol (20:1) als Eluens chromatographiert.
Ausbeute: 828 mg (88%)
$R_f$ (Dichlormethan/Methanol 20.1) = 0,27
Ein Teil wird wie unter Beispiel 1 beschrieben in das Trihydrochlorid überführt

## Beispiel 3

1-[4,4-Bis-(4-fluorphenyl)butyl]-4-[2-(2-N,N-dimethylaminophenoxy)ethyl]piperazin

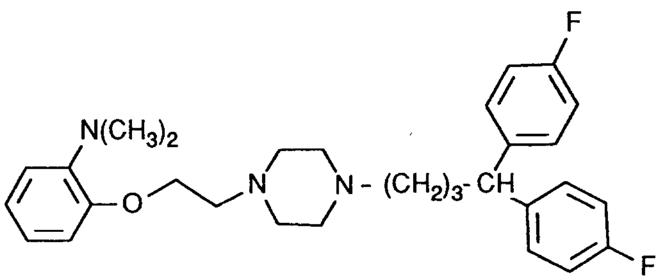

121 mg (0,26 mmol) des Anilins aus Beispiel 2 werden in 1,8 ml Tetrahydrofuran mit 69 mg (1,82 mmol) Natriumborhydrid versetzt und auf 0 °C gekühlt. Dann fügt man eine Lösung aus 195 µl 3 M Schwefelsäure und 110 µl (1,56 mmol) 35% Formaldehyd in Wasser zu, stellt mit 2 N Natronlauge alkalisch und extrahiert 3 mal mit Essigester. Die vereinigten organischen Phasen werden getrocknet ($MgSO_4$) und eingeengt, und der Rückstand wird dann an Kieselgel mit Dichlormethan/Methanol (50:1) chromatographiert.
Ausbeute: 53 mg (41%)

**Beispiel 4**

1-[4,4-Bis-(4-fluorphenyl)butyl]-4-[2-(2-acetylaminophenoxy)ethyl]piperazin

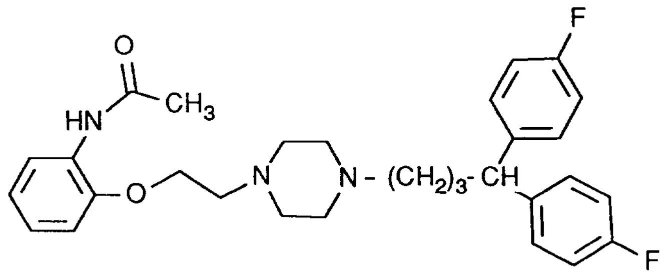

415 mg (0.89 mmol) des Anilins aus Beispiel 2 werden in 3,2 ml Dichlormethan bei 0 °C mit 222 µl Triethylamin und 114 µl Acetylchlorid versetzt. Nach 10 min wird mit ges. NaCl-Lösung versetzt und 3 mal mit Dichlormethan extrahiert. Die vereinigten organischen Phasen werden getrocknet ($MgSO_4$) und eingeengt und der Rückstand an Kieselgel mit Dichlormethan/Methanol (50:1) chromatographiert.
Ausbeute: 401 mg (88,5%)
$R_f$ (Dichlormethan/Methanol 20:1) = 0,56

**Beispiel 5**

1-[4,4-Bis-(4-fluorphenyl)butyl]-4-[2-(N-methyl-acetylaminophenoxy)ethyl]piperazin

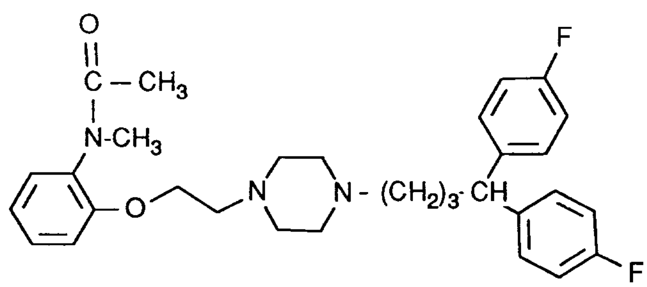

389 mg (0,77 mmol) des Acetanilids aus Beispiel 4 in 5,8 ml Dimethylformamid werden bei -20 °C mit 30 mg (1,0 mmol) 80% NaH-Suspension und 275 mg (1,0 mmol) Methyljodid versetzt. Nach 10 min wird mit Wasser verdünnt, 3 mal mit Ether extrahiert, und die vereinigten organischen Phasen werden getrocknet ($MgSO_4$) und eingeengt. Der Rückstand wird an Kieselgel mit Dichlormethan/Methanol (20:1) chromatographiert.
Ausbeute: 124 mg (31%)

**Beispiel 6**

1-[4,4-Bis-(4-fluorphenyl)butyl]-4-[2-(2-N,N-ethylmethylaminophenoxy)ethyl]piperazin

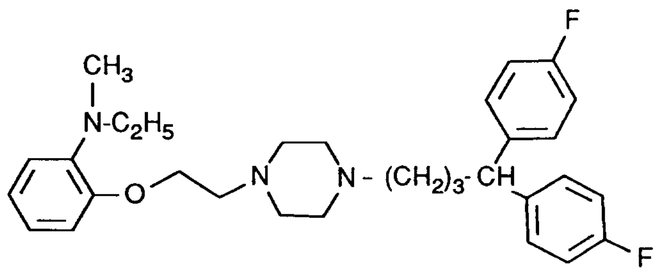

103 mg (0,2 mmol) der Verbindung aus Beispiel 5 in 1,5 ml Ether werden mit 15 mg (0,4 mmol) Lithiumaluminiumhydrid 1 h bei RT gerührt. Dann wird Wasser zugefügt, mit Schwefelsäure sauer gestellt, mit Essigester gewaschen, die wäßrige Phase mit 2 N Natronlauge basisch gestellt und 3 mal mit Essigester extrahiert. Die verenigten organischen Phasen werden getrocknet ($MgSO_4$) und eingeengt und der Rückstand mit Dichlormethan/Methanol (20:1) als Eluens chromatographiert.
Ausbeute: 63 mg (63%)

**Beispiel 7**

1-[4,4-Bis-(4-fluorphenyl)butyl]-4-[2-(4-chlor-2-hydroxycarbonylphenoxy)ethyl]piperazin

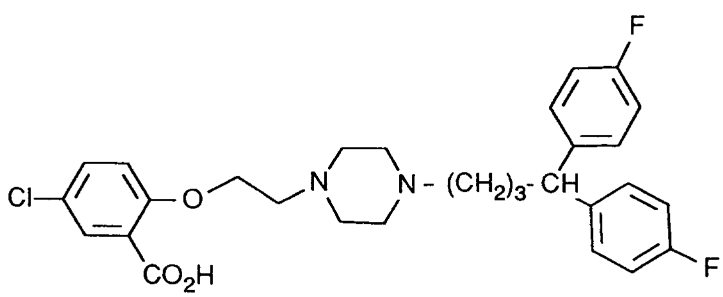

Ausgehend von 5-Chlor-2-hydroxybenzoesäuremethylester wird, wie unter Beispiel 1 beschrieben, 1-[4,4-Bis-(4-fluorphenyl)butyl]-4-[2-(4-chlor-2-methoxycarbonylphenoxy)ethyl]piperazin dargestellt. 8,3 g (15,3 mmol) dieser Verbindung werden in 100 ml Methanol und 15 ml Wasser in Gegenwart von 1,1 g (20 mmol) Kaliumhydroxid 18 h bei RT gerührt. Dann wird eingeengt, zwischen Essigester und Wasser verteilt, und das Gemisch mit 6 N Salzsäure auf pH 2 gestellt. Die organische Phase wird abgetrennt, getrocknet ($MgSO_4$) und auf 50 ml eingeengt. Nach Versetzen mit Ether wird ein Feststoff erhalten, der abgesaugt und getrocknet wird.
Ausbeute: 6,5 g (80%)
$R_f$ (Dichlormethan/Ethanol (5:1)) = 0,13

**Beispiel 8**

1-[4,4-Bis-(4-fluorphenyl)butyl]-4-[2-[4-chlor-2-(N-((1S)-1-tert.butoxycarbonyl-2-methylpropyl)aminocarbonyl)-phenoxy]ethyl]piperazin

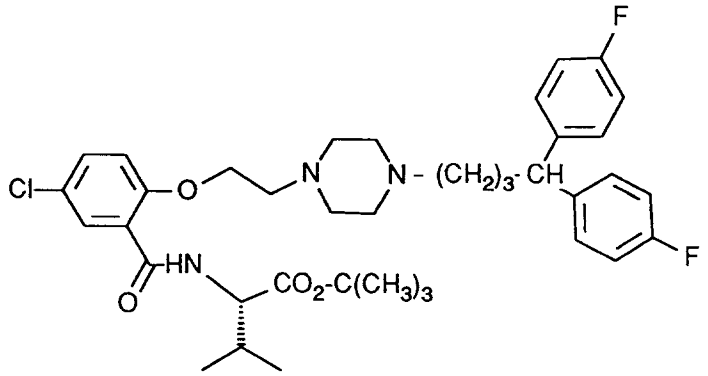

529 mg (1 mmol) der Säure aus Beispiel 7 und 174 mg (1 mmol) L-Valin-tert.butylester werden nach der Methode aus Beispiel II gekuppelt. Reinigung erfolgt durch Chromatographie an Kieselgel mit Dichlormethan/Ethanol (30:1) als Eluens.
Ausbeute: 630 mg (92%)
$R_f$ (Dichlormethan/Ethanol 20:1) = 0,45

**Beispiel 9**

1-[4,4-Bis-(4-fluorphenyl)butyl]-4-[2-[4-chlor-2-(N-((1S)-1-hydroxycarbonyl-2-methylpropyl)aminocarbonyl)-phenoxy]ethyl]piperazin

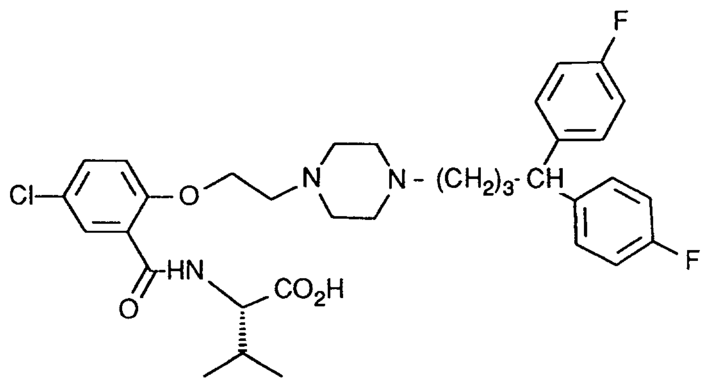

360 mg (0,53 mmol) des Esters aus Beispiel 8 werden bei 0°C in 1 ml Dichlormethan zu 1 ml Trifluoressigsäure gegeben. Man läßt 18 h bei RT stehen, engt dann ein und trocknet den Rückstand. Anschließend wird dieser in Dichlormethan aufgenommen und mit Ether/Petrolether versetzt. Man läßt absitzen, dekantiert das Lösemittel ab und trocknet den Rückstand.
Ausbeute: 310 mg (94%)

**Beispiel 10**

1-[4,4-Bis-(4-fluorphenyl)butyl]-4-[(R/S)-2-(2,4-difluorphenoxy)propyl]piperazin

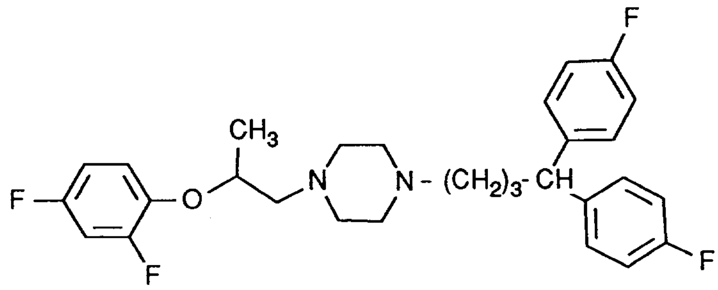

Die Titelverbindung wird dargestellt durch Verknüpfung von 2,4-Difluorphenol mit (R/S)-2-Brompropionsäureethylester nach der Methode von Beispiel I und weitere Umwandlung des erhaltenen Esters analog Beispiel 1.
$R_f$ (Essigester/Aceton 10:1) = 0,56

**Beispiel 11**

1-[4,4-Bis-(4-fluorphenyl)butyl]-4-[(2R/S)-2-(2,4-difluorphenoxy)-2-ethoxycarbonylethyl]piperazin

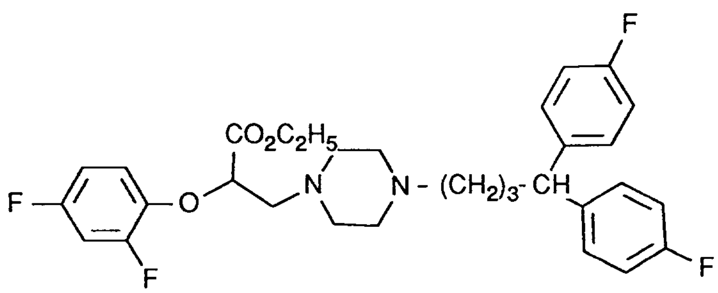

Die Carbonsäure aus Beispiel IV wird wie für Beispiel II beschrieben zum Amid umgesetzt. 32,2 g (61 mmol) des Amides in 200 ml Tetrahydrofuran (THF) werden unter Eiskühlung mit 135 ml einer 2 M Lösung von Boran-Dimethylsulfid in THF versetzt und 8 h bei RT gehalten. Dann wird unter Eiskühlung mit 70 ml 15%iger Salzsäure versetzt, 20 min auf 50 °C erwärmt und mit Natronlauge pH 10 eingestellt. Es wird 2 mal mit Essigester extrahiert, getrocknet ($MgSO_4$) und eingeengt. Der Rückstand wird an Kieselgel mit Dichlormethan/Ethanol (20.1) als Eluens chromatographiert.
Ausbeute: 19,5 g (57%)
$R_f$ (Dichlormethan/Ethanol 15:1) = 0,6

**Beispiel 12**

1-[4,4-Bis-(4-fluorphenyl)butyl]-4-[((2R/S)-2-(2,4-difluorphenoxy)-2-(N-(1S)-1-tert.butoxycarbonyl-2-methylpropyl)aminocarbonyl)ethyl]piperazin

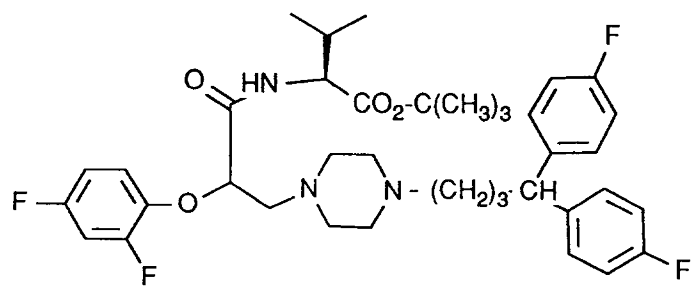

Der Ester aus Beispiel 11 wird wie unter den Beispielen 7 und 8 beschrieben, verseift und mit (L)-Valin-tert.butylester zum Amid umgesetzt.
$R_f$ (Dichlormethan/Ethanol 15:1) = 0,52 (1.Diastereomer) 0,38 (2. Diastereomer)

**Beispiel 13**

(S)-1-[4,4-Bis-(4-fluorphenyl)butyl]-2-methyl-4-[2-(2,4-difluorphenoxy)ethyl]piperazin

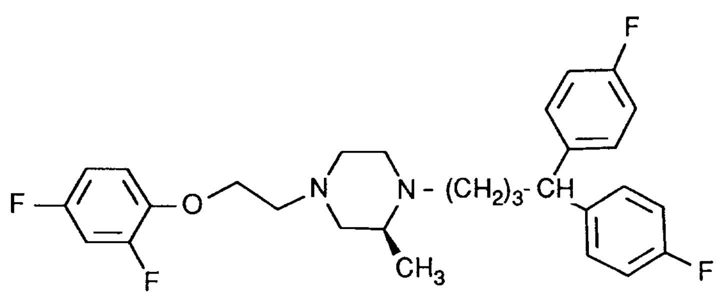

Das Piperazin aus Beispiel V wird analog zu Beispiel II mit 2,4-Difluorphenoxyessigsäure (hergestellt aus 2,4-Difluorphenol und Bromessigsäure-tert.butylester analog Beispiel I) zum Anhydrid gekuppelt; dann werden beide Amidgruppen des Zwischenproduktes analog zu Beispiel 1 unter Einsatz von 10 eq Boran in Tetrahydrofuran reduziert.
Ausbeute: 85% (über beide Schritte)
MS(FAB): m/z = 501 ($M^+$ + H)
$R_f$ (Dichlormethan/Ethanol 9:1) = 0,51

**Beispiel 14**

(S)-4-[4,4-Bis-(4-fluorphenyl)butyl]-2-methyl-1-[2-(2,4-difluorphenoxy)ethyl]piperazin

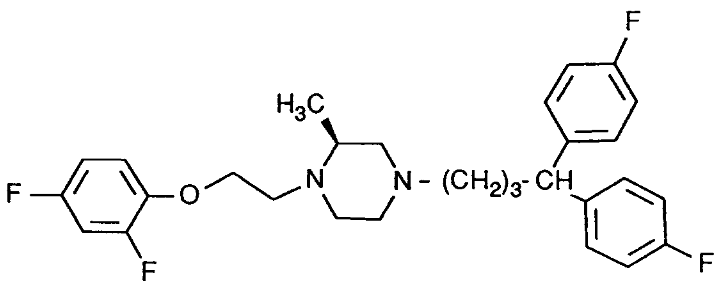

2,4-Difluorphenoxyessigsäure (hergestellt aus 2,4-Difluorphenol und Bromessigsäure-tert.butylester analog Beispiel I) und (S)-1-Benzyl-3-methylpiperazin (siehe Beispiel V) werden analog zu Beispiel II zum Amid umgesetzt. Anschließend wird die Benzylgruppe wie unter Beispiel V gezeigt abgespalten, das Amid mit 4,4-Bis-(4-fluorphenyl)butansäure gebildet (siehe Beispiel V), und zum Schluß werden beide Amidgruppen, wie unter Beispiel 13 gezeigt, reduziert.

MS(FAB): m/z = 501 ($M^+ + H$)

$R_f$ (Dichlormethan/Methanol 9:1) = 0,61

In Analogie zur Vorschrift des Beispiels 4 werden die in den Tabellen 1, 2 und 3 aufgeführten Verbindungen hergestellt:

Tabelle 1:

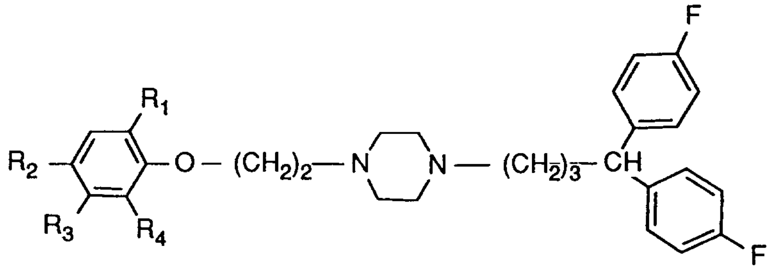

| Bsp.-Nr. | $R^1$ | $R^2$ | $R^3$ | $R^4$ | $R_f$ *) |
|---|---|---|---|---|---|
| 15 | H | F | H | - F | 0,53 / I |
| 16 | H | H | $-CH_3$ | $-CH_3$ | 0,59 / I |
| 17 | H | Cl | H | $-CH_3$ | 0,57 / I |
| 18 | H | H | H | $-NO_2$ | 0,29 / III |
| 19 | H | H | H | -OH | 0,18 / III |
| 20 | H | H | Cl | -Cl | 0,32 / III |
| 21 | H | -CHO | H | $-OCH_3$ | 0,4 / IV |
| 22 | H | $-CH_3$ | H | $-OCH_3$ | 0,57 / I |
| 23 | $- C_6H_5$ | $-NO_2$ | H | H | 0,16 / III |
| 24 | H | H | H | $-C_6H_5$ | 0,19 / III |
| 25 | H | H | H | - F | 0,53 / I |

Tabelle 2:

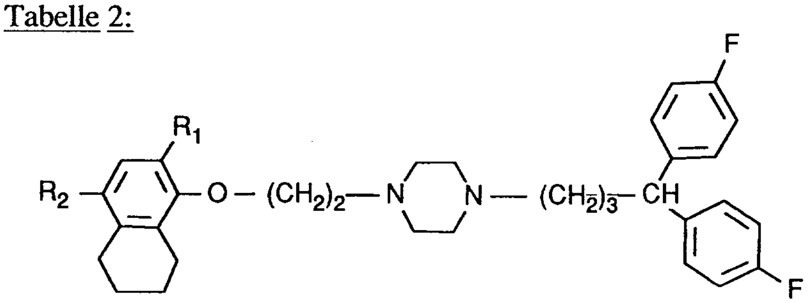

| Bsp.-Nr. | $R^1$ | $R^2$ | $R_f$ *) |
|---|---|---|---|
| 26 | H | H | 0,62 / I |

Tabelle 3:

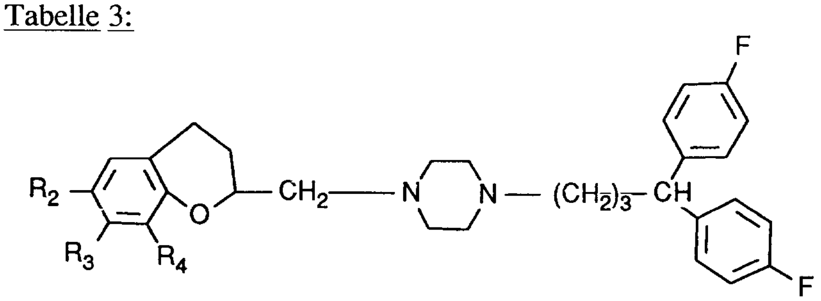

| Bsp.-Nr. | $R^2$ | $R^3$ | $R^4$ | $R_f$ *) |
|---|---|---|---|---|
| 27 | H | H | $OCH_3$ | 0,53 / II |
| 28 | H | H | H | 0,57 / II |

In Analogie zu den Vorschriften der Beispiele 2 - 6 werden die in Tabelle 4 aufgeführten Verbindungen hergestellt:

Tabelle 4:

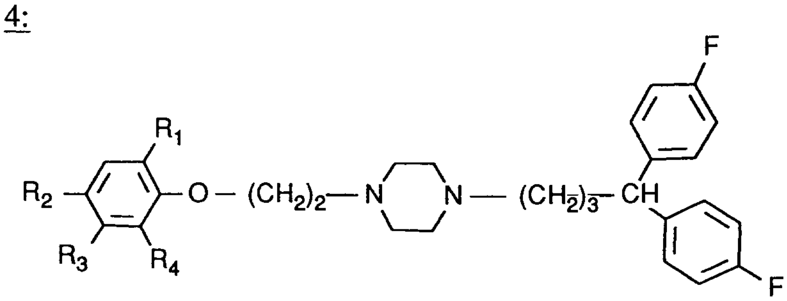

| Bsp.-Nr. | $R^1$ | $R^2$ | $R^3$ | $R^4$ | $R_f$ *) |
|---|---|---|---|---|---|
| 29 | H | H | $-NH_2$ | H | 0,49 / V |
| 30 | $-CH_3$ | H | $-N(CH_3)_2$ | H | 0,83 / V |
| 31 | H | -Cl | H | $NH_2$ | 0,32 / V |
| 32 | $-CH_3$ | H | $-NH_2$ | H | 0,53 / V |
| 33 | H | $-CO_2CH_3$ | H | $-N(CH_3)-CO-CH_3$ | 0,30 / VI |
| 34 | H | $-CH_2OH$ | H | $-N(CH_3)-C_2H_5$ | 0,30 / VI |

In Analogie zu den Vorschriften der Beispiele 7 - 9 werden die in Tabelle 5 aufgeführten Verbindungen hergestellt:

Tabelle 5:

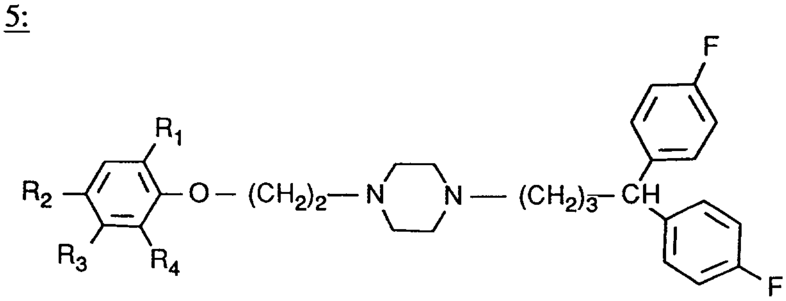

| Bsp.-Nr. | $R^1$ | $R^2$ | $R^3$ | $R^4$ | $R_f$ *) |
|---|---|---|---|---|---|
| 35 | H | H | H | $-CONH_2$ | 0,27 / V |
| 36 | H | -Cl | H | $-CONHCH_2Ph$ | 0,36 / V |

In Analogie zur Vorschrift des Beispiels 10 werden die in Tabelle 6 aufgeführten Verbindungen hergestellt:

Tabelle 6:

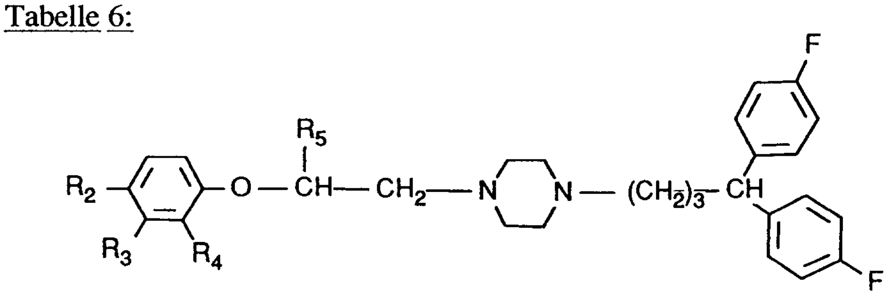

| Bsp.-Nr. | $R^2$ | $R^3$ | $R^4$ | $R^5$ | $R_f$ *) |
|---|---|---|---|---|---|
| 37 | H | $-CH_3$ | $-CH_3$ | $-CH_3$ | 0,59 / I |
| 38 | H | $-CF_3$ | H | $-CH_3$ | 0,59 / I |
| 39 | -Cl | H | $-CH_3$ | $-CH_3$ | 0,63 / I |
| 40 | $-CF_3$ | H | H | $-CH_3$ | 0,60 / I |
| 41 | H | $-(CH_2)_4-$ | | $-CH_3$ | 0,66 / I |

In Analogie zu den Vorschriften der Beispiele 11 und 12 werden die in Tabelle 7 aufgeführten Verbindungen hergestellt:

Tabelle 7:

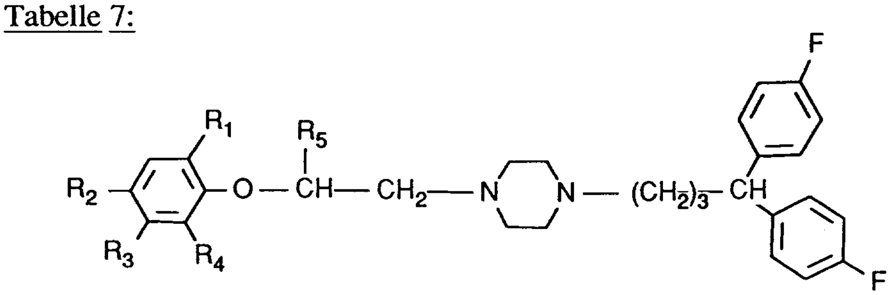

| Bsp.-Nr. | $R^1$ | $R^2$ | $R^3$ | $R^4$ | $R^5$ | $R_f$ *) |
|---|---|---|---|---|---|---|
| 42 | H | -F | H | -F | $-CONHCH_2Ph$ | 0,20 / II |

In Analogie zur Vorschrift des Beispiels 13 werden die in Tabelle 8 aufgeführten Verbindungen hergestellt:

Tabelle 8:

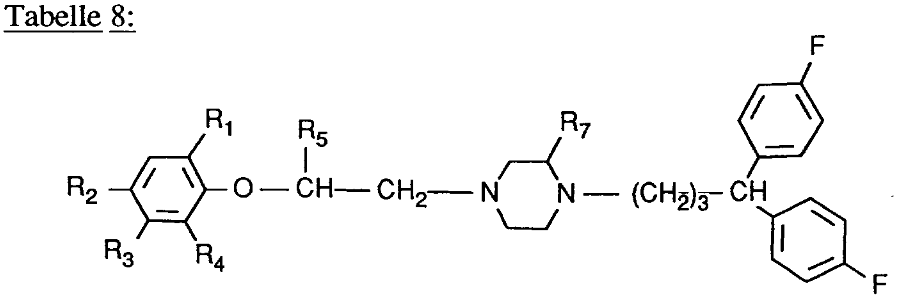

| Bsp.-Nr. | $R^1$ | $R^2$ | $R^3$ | $R^4$ | $R^5$ | $R^7$ | $R_f$ *) |
|---|---|---|---|---|---|---|---|
| 43 | H | -F | H | -F | H | (R)-$CH_2OH$ | 0,47 / II |

In Analogie zur Vorschrift des Beispiels 14 werden die in Tabelle 9 aufgeführten Verbindungen hergestellt:

Tabelle 9:

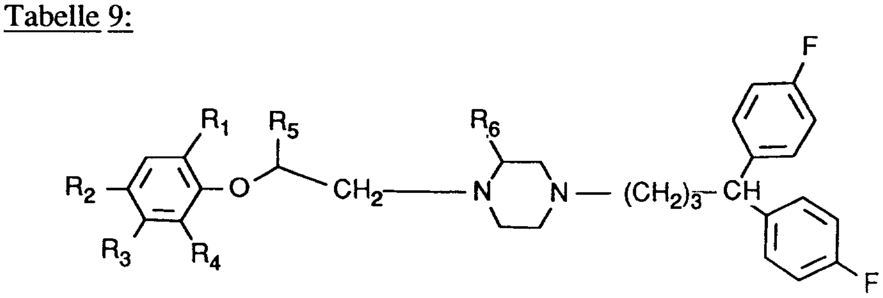

| Bsp.-Nr. | $R^2$ | $R^3$ | $R^4$ | $R^1$ | $R^5$ | $R^6$ | $R_f$ *) |
|---|---|---|---|---|---|---|---|
| 44 | H | H | $OCH_3$ | -$(CH_2)_2$- | | (S)-$CH_3$ | 0,57 / II |
| 45 | -F | H | -F | H | H | (R)-$CH_2OH$ | 0,45 / II |
| 46 | -F | H | -F | H | H | (S)-$CH_2$-$C_6H_5$ | 0,72 / II |

*) $R_f$-Werte in Lösemittelgemischen:

I = Essigester/Aceton 10:1
II = Dichlormethan/Methanol 9:1
III = Dichlormethan/Methanol 50:1
IV = Essigester/Aceton 1:1
V = Dichlormethan/Methanol 20.1
VI = Aceton

## Patentansprüche

1. Substituierte Piperazine der allgemeinen Formel (I)

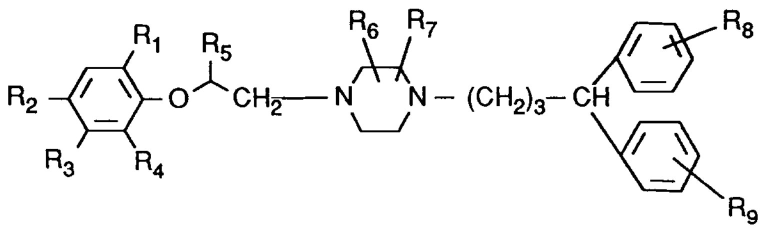

(I)

in welcher

| | |
|---|---|
| $R^1$, $R^2$, $R^3$ und $R^4$ | gleich oder verschieden sind und für Wasserstoff, Nitro, Halogen, Carboxy, Hydroxy, Formyl, geradkettiges oder verzweigtes Alkoxy oder Alkoxycarbonyl mit jeweils bis zu 8 Kohlenstoffatomen, Trifluormethyl, Phenyl oder<br>für geradkettiges oder verzweigtes Alkyl mit bis zu 8 Kohlenstoffatomen stehen, das gegebenenfalls durch Hydroxy oder geschütztes Hydroxy substituiert ist, oder<br>für eine Gruppe der Formel $-NR^{10}R^{11}$ oder -CO-A stehen,<br>worin |
| $R^{10}$ und $R^{11}$ | gleich oder verschieden sind und Wasserstoff, Phenyl, Benzyl, eine Aminoschutzgruppe oder geradkettiges oder verzweigtes Alkyl mit bis zu 8 Kohlenstoffatomen bedeuten,<br>oder |
| $R^{10}$ und $R^{11}$ | gemeinsam mit dem Stickstoffatom einen 5- bis 7-gliedrigen, gesättigten Heterocyclus bilden, |
| A | die oben angegebene Gruppe $-NR^{10}R^{11}$ oder den Rest der Formel |

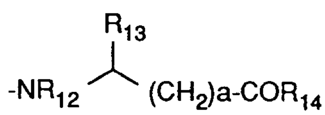

| | |
|---|---|
| | bedeutet,<br>worin |
| $R^{10}$ und $R^{11}$ | die oben angegebene Bedeutung haben, |
| $R^{12}$ | Wasserstoff oder geradkettiges oder verzweigtes Alkyl mit bis zu 4 Kohlenstoffatomen bedeutet, |
| $R^{13}$ | Cycloalkyl mit 3 bis 8 Kohlenstoffatomen oder Aryl mit 6 bis 10 Kohlenstoffatomen oder Wasserstoff bedeutet, oder<br>geradkettiges oder verzweigtes Alkyl mit bis zu 8 Kohlenstoffatomen bedeutet,<br>wobei das Alkyl gegebenenfalls durch Methylthio, Hydroxy, Mercapto, Guanidyl oder durch eine Gruppe der Formel $-NR^{15}R^{16}$ oder $R^{17}$-OC- substituiert ist,<br>worin |
| $R^{15}$ und $R^{16}$ | unabhängig voneinander Wasserstoff, geradkettiges oder verzweigtes Alkyl mit bis zu 8 Kohlenstoffatomen oder Phenyl bedeuten,<br>und |
| $R^{17}$ | Hydroxy, Benzyloxy, Alkoxy mit bis zu 6 Kohlenstoffatomen oder die oben aufgeführte Gruppe $-NR^{15}R^{16}$ bedeutet,<br>worin |
| $R^{15}$ und $R^{16}$ | die oben angegebene Bedeutung haben, |

oder das Alkyl gegebenenfalls durch Cycloalkyl mit 3 bis 8 Kohlenstoffatomen oder durch Aryl mit 6 bis 10 Kohlenstoffatomen substituiert ist, das seinerseits durch Hydroxy, Halogen, Nitro, Alkoxy mit bis zu 8 Kohlenstoffatomen oder durch die Gruppe $-NR^{15}R^{16}$ substituiert ist,
worin

| | |
|---|---|
| $R^{15}$ und $R^{16}$ | die oben angegebene Bedeutung haben, oder das Alkyl gegebenenfalls durch einen 5- bis 6-gliedrigen stickstoffhaltigen Heterocyclus oder Indolyl substituiert ist, worin die entsprechenden -NH-Funktionen gegebenenfalls durch Alkyl mit bis zu 6 Kohlenstoffatomen oder durch eine Aminoschutzgruppe geschützt sind, und |
| a | die Zahl 0 oder 1 bedeutet, |
| $R^{14}$ | Hydroxy oder geradkettiges oder verzweigtes Alkoxy mit bis zu 8 Kohlenstoffatomen bedeutet, oder |
| $R^2$ und $R^3$ oder $R^3$ und $R^4$ | gemeinsam einen unter Einbezug der phenylischen Doppelbindung, partiell ungesättigten 5- bis 7-gliedrigen Carbocyclus bilden, |
| $R^5$ | für Wasserstoff oder für geradkettiges oder verzweigtes Alkyl mit bis zu 8 Kohlenstoffatomen steht, oder für eine Gruppe der Formel $-CO_2R^{18}$ steht, worin |
| $R^{18}$ | Wasserstoff, geradkettiges oder verzweigtes Alkyl mit bis zu 8 Kohlenstoffatomen oder eine Hydroxyschutzgruppe bedeutet, oder |
| $R^5$ | für eine Gruppe der Formel -CO-B steht, worin |
| B | die oben angegebene Bedeutung von A hat und mit dieser gleich oder verschieden ist, oder |
| $R^1$ und $R^5$ | unter Einbezug des Sauerstoffatoms und des Phenylrings zusammen einen 5- bis 6-gliedrigen, ungesättigten Heterocyclus bilden, |
| $R^6$ und $R^7$ | gleich oder verschieden sind und für Wasserstoff, Carboxy oder geradkettiges oder verzweigtes Alkyl mit bis zu 8 Kohlenstoffatomen stehen, das gegebenenfalls durch Hydroxy oder Phenyl substituiert ist, oder für geradkettiges oder verzweigtes Alkoxycarbonyl mit bis zu 8 Kohlenstoffatomen stehen, |
| $R^8$ und $R^9$ | gleich oder verschieden sind und für Halogen, Cyano, Nitro, Azido oder Hydroxy stehen, |

und deren Salze.

**2.** Verbindungen der allgemeinen Formel (I), gemäß Anspruch 1 in welcher

| | |
|---|---|
| $R^1$, $R^2$, $R^3$ und $R^4$ | gleich oder verschieden sind und für Wasserstoff, Nitro, Fluor, Chlor, Brom, Carboxy, Hydroxy, Formyl, geradkettiges oder verzweigtes Alkoxy oder Alkoxycarbonyl mit jeweils bis zu 6 Kohlenstoffatomen, Trifluormethyl, Phenyl oder für geradkettiges oder verzweigtes Alkyl mit bis zu 6 Kohlenstoffatomen stehen, das gegebenenfalls durch Hydroxy oder geschütztes Hydroxy substituiert ist, oder für eine Gruppe der Formel $-NR^{10}R^{11}$ oder -CO-A stehen, worin |
| $R^{10}$ und $R^{11}$ | gleich oder verschieden sind und Wasserstoff, Phenyl, Benzyloxycarbonyl, Butyl, tert.Butyloxycarbonyl (BOC), Acetyl, Benzyl oder geradkettiges oder verzweigtes Alkyl mit bis zu 6 Kohlenstoffatomen bedeuten, |

oder

$R^{10}$ und $R^{11}$ gemeinsam mit dem Stickstoffatom einen Pyrrolidin- oder Piperidinring bilden,

A die oben angegebene Gruppe $-NR^{10}R^{11}$ oder den Rest der Formel

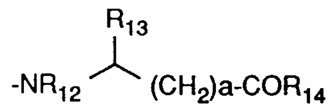

bedeutet,

worin

$R^{10}$ und $R^{11}$ die oben angegebene Bedeutung haben,

$R^{12}$ Wasserstoff, Methyl oder Ethyl bedeutet,

$R^{13}$ Cyclopentyl, Cyclohexyl, Phenyl oder Wasserstoff bedeutet, oder geradkettiges oder verzweigtes Alkyl mit bis zu 6 Kohlenstoffatomen bedeutet,

wobei das Alkyl gegebenenfalls durch Methylthio, Hydroxy, Mercapto, Guanidyl, Amino, Carboxy oder $H_2N$-CO-substituiert sein kann,

oder das Alkyl durch Cyclohexyl, Naphthyl oder Phenyl substituiert ist, das seinerseits durch Fluor, Hydroxy, Nitro oder Alkoxy mit bis zu 4 Kohlenstoffatomen substituiert sein kann,

oder das Alkyl durch Indolyl, Imidazolyl, Pyridyl, Triazolyl oder Pyrazolyl substituiert ist, wobei die entsprechenden -NH-Funktionen gegebenenfalls durch Alkyl mit bis zu 4 Kohlenstoffatomen oder durch eine Aminoschutzgruppe geschützt sind,

$R^{14}$ Hydroxy oder geradkettiges oder verzweigtes Alkoxy mit bis zu 6 Kohlenstofatomen bedeutet,

a die Zahl 0 oder 1 bedeutet,

oder

$R^2$ und $R^3$ oder $R^3$ und $R^4$ jeweils gemeinsam, unter Einbezug der phenylischen Doppelbindung, einen Cyclohexenylring bilden,

$R^5$ für Wasserstoff oder geradkettiges oder verzweigtes Alkyl mit bis zu 6 Kohlenstoffatomen steht, oder

für eine Gruppe der Formel $-CO_2R^{18}$ steht,

worin

$R^{18}$ Wasserstoff, geradkettiges oder verzweigtes Alkyl mit bis zu 6 Kohlenstoffatomen, Acetyl oder Benzyl bedeutet,

oder

$R^5$ für eine Gruppe der Formel -CO-B steht,

worin

B die oben angegebene Bedeutung von A hat und mit dieser gleich oder verschieden ist,

oder

$R^1$ und $R^5$ unter Einbezug des Sauerstoffatoms und des Phenylrings zusammen einen Chromanring bilden,

$R^6$ und $R^7$ gleich oder verschieden sind und für Wasserstoff, Carboxy oder geradkettiges oder verzweigtes Alkyl mit bis zu 6 Kohlenstoffatomen stehen, das gegebenenfalls durch Hydroxy oder Phenyl substituiert ist, oder

für geradkettiges oder verzweigtes Alkoxycarbonyl mit bis zu 6 Kohlenstoffatomen stehen,

$R^8$ und $R^9$ gleich oder verschieden sind und für Fluor, Chlor, Cyano, Nitro, Azido oder Hydroxy stehen,

und deren Salze.

**3.** Verbindungen der allgemeinen Formel (I) gemäß Anspruch 1 in welcher

$R^1$, $R^2$, $R^3$ und $R^4$ gleich oder verschieden sind und für Wasserstoff, Nitro, Fluor, Chlor, Brom, Carboxy, Hydroxy, Formyl, geradkettiges oder verzweigtes Alkoxy oder Alkoxycarbonyl mit jeweils bis zu 4 Kohlenstoffatomen, Trifluormethyl, Phenyl oder für geradkettiges oder verzweigtes Alkyl mit bis zu 4 Kohlenstoffatomen stehen, das gegebenenfalls durch Hydroxy substituiert ist, oder für eine Gruppe der Formel $-NR^{10}R^{11}$ oder -CO-A stehen,

worin

$R^{10}$ und $R^{11}$ gleich oder verschieden sind und Wasserstoff, Phenyl, Benzyl, Benzyloxycarbonyl, Butyl, tert.Butyloxycarbonyl (BOC), Acetyl oder geradkettiges oder verzweigtes Alkyl mit bis zu 4 Kohlenstoffatomen bedeuten,

oder

$R^{10}$ und $R^{11}$ gemeinsam mit dem Stickstoffatom einen Pyrrolidin- oder Piperidinring bilden,

A die oben angegebene Gruppe $-NR^{10}R^{11}$ oder den Rest der Formel

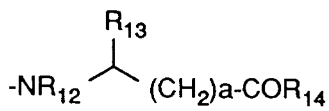

bedeutet,

worin

$R^{10}$ und $R^{11}$ die oben angegebene Bedeutung haben,

$R^{12}$ Wasserstoff, Methyl oder Ethyl bedeutet,

$R^{13}$ Cyclopentyl, Cyclohexyl, Phenyl oder Wasserstoff bedeutet, oder geradkettiges oder verzweigtes Alkyl mit bis zu 4 Kohlenstoffatomen bedeutet,

wobei das Alkyl gegebenenfalls durch Methylthio, Hydroxy, Mercapto, Guanidyl, Amino, Carboxy oder $H_2N$-CO- substituiert sein kann,

oder das Alkyl durch Cyclohexyl, Naphthyl oder Phenyl substituiert ist, das seinerseits durch Fluor, Hydroxy, Nitro oder Alkoxy mit bis zu 4 Kohlenstoffatomen substituiert sein kann,

oder das Alkyl durch Indolyl, Imidazolyl, Pyridyl, Triazolyl oder Pyrazolyl substituiert ist, wobei die entsprechenden -NH-Funktionen gegebenenfalls durch Alkyl mit bis zu 4 Kohlenstoffatomen oder durch eine Aminoschutzgruppe geschützt sind,

$R^{14}$ Hydroxy oder geradkettiges oder verzweigtes Alkoxy mit bis zu 4 Kohlenstofatomen bedeutet,

a die Zahl 0 oder 1 bedeutet,

oder

$R^2$ und $R^3$ oder $R^3$ und $R^4$ jeweils gemeinsam, unter Einbezug der phenylischen Doppelbindung, einen Cyclohexenylring bilden,

$R^5$ für Wasserstoff oder geradkettiges oder verzweigtes Alkyl mit bis zu 4 Kohlenstoffatomen steht, oder für eine Gruppe der Formel $-CO_2R^{18}$ steht,

worin

$R^{18}$ Wasserstoff, geradkettiges oder verzweigtes Alkyl mit bis zu 4 Kohlenstoffaotmen, Acetyl oder Benzyl bedeutet,

oder

$R^5$ für eine Gruppe der Formel -CO-B steht,

worin

B die oben angegebene Bedeutung von A hat und mit dieser gleich oder verschieden ist,

oder

$R^1$ und $R^5$ unter Einbezug des Sauerstoffatoms und des Phenylrings zusammen einen Chromanring bilden,

$R^6$ und $R^7$ gleich oder verschieden sind und für Wasserstoff, Carboxy oder geradkettiges oder verzweigtes Alkyl mit bis zu 4 Kohlenstoffatomen stehen, das gegebenenfalls durch Hydroxy oder Phenyl substituiert ist, oder für geradkettiges oder verzweigtes Alkoxycarbonyl mit bis zu 4 Kohlenstoffatomen stehen,

$R^8$ und $R^9$ gleich oder verschieden sind und für Fluor, Chlor, Cyano, Nitro oder Hydroxy stehen,

und deren Salze.

**4.** Verfahren zur Herstellung der Verbindungen der allgemeinen Formel (I) gemäß Anspruch 1, dadurch gekennzeichnet, daß man

[A] Verbindungen der allgemeinen Formel (II)

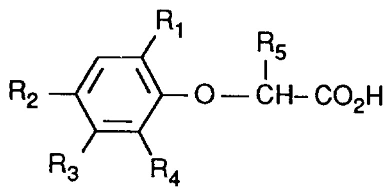

(II)

in welcher

$R^1$, $R^2$, $R^3$, $R^4$ und $R^5$ die oben angegebene Bedeutung haben,

zunächst durch Umsetzung mit Verbindungen der allgemeinen Formel (III)

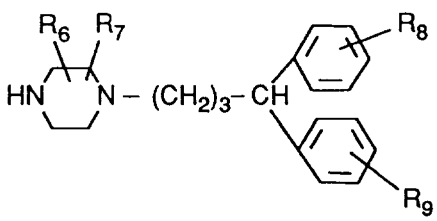

(III)

in welcher

$R^6$, $R^7$, $R^8$ und $R^9$ die in Anspruch 1 angegebene Bedeutung haben,

in inerten Lösemitteln, in Anwesenheit einer Base und/oder Hilfsstoffes, gegebenenfalls unter vorgeschalteter Aktivierung der Carbonsäurefunktion,

in die Verbindungen der allgemeinen Formel (IV)

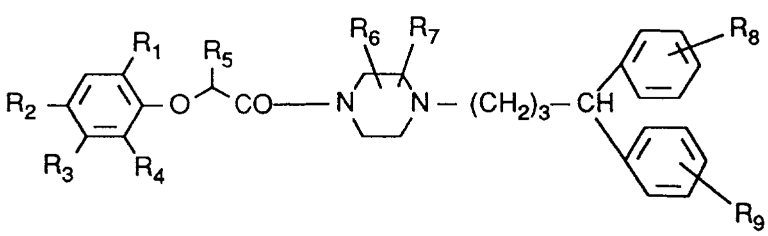

(IV)

in welcher

$R^1$, $R^2$, $R^3$, $R^4$, $R^5$, $R^6$, $R^7$, $R^8$ und $R^9$ die in Anspruch 1 angegebene Bedeutung haben,

überführt und in einem zweiten Schritt nach üblichen Methoden die Carbonylfunktion reduziert.

**5.** Verfahren zur Herstellung von Verbindungen der Formel (I) gemäß Anspruch 1, dadurch gekennzeichnet, daß man Verbindungen der allgemeinen Formel (V)

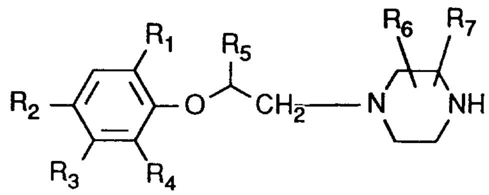

(V)

in welcher

$R^1$, $R^2$, $R^3$, $R^4$, $R^5$, $R^6$ und $R^7$ die in Anspruch 1 angegebene Bedeutung haben,

zunächst durch Umsetzung mit Verbindungen der allgemeinen Formel (VI)

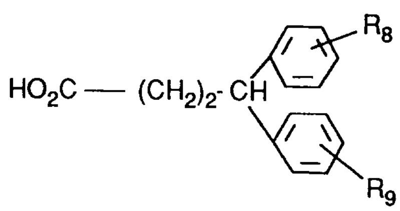

(VI)

in welcher

$R^8$ und $R^9$ die in Anspruch 1 angegebene Bedeutung haben,

in inerten Lösemitteln, gegebenenfalls in Anwesenheit einer Base und/oder Hilfsstoffes, in die Verbindungen der allgemeinen Formel (VII)

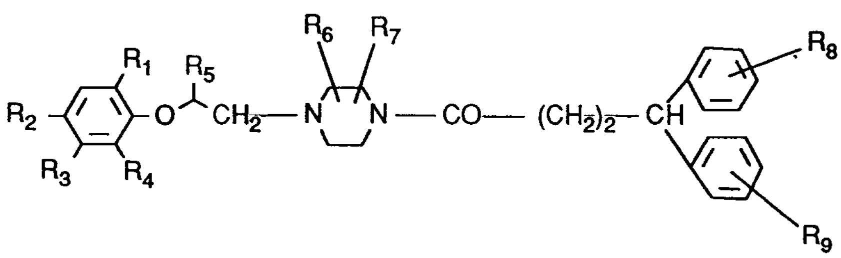

(VII)

in welcher

$R^1$, $R^2$, $R^3$, $R^4$, $R^5$, $R^6$, $R^7$, $R^8$ und $R^9$ die in Anspruch 1 angegebene Bedeutung haben,

überführt, und anschließend die Carbonylfunktion reduziert.

**6.** Verfahren zur Herstellung von Verbindungen der Formel (I) gemäß Anspruch 1, dadurch gekennzeichnet, daß man Verbindungen der allgemeinen Formel (VIII)

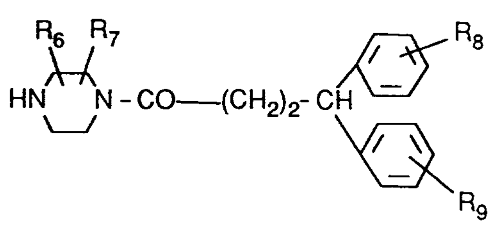

(VIII)

in welcher

$R^6$, $R^7$, $R^8$ und $R^9$ die in Anspruch 1 angegebene Bedeutung haben,

zunächst durch Umsetzung mit Verbindungen der allgemeinen Formel (II), wie unter [A] beschrieben, in die Verbindungen der allgemeinen Formel (IX)

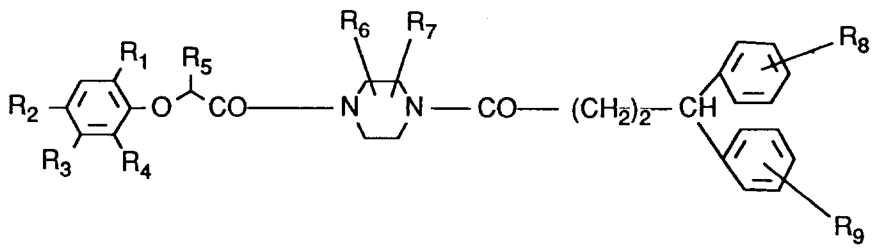

(IX)

in welcher

$R^1$, $R^2$, $R^3$, $R^4$, $R^5$, $R^6$, $R^7$, $R^8$ und $R^9$ die oben angegebene Bedeutung haben,

überführt und anschließend die Carbonylfunktion reduziert.

**7.** Verfahren zur Herstellung von Verbindungen der Formel (I) gemäß Anspruch 1, dadurch gekennzeichnet, daß man Verbindungen der allgemeinen Formel (X)

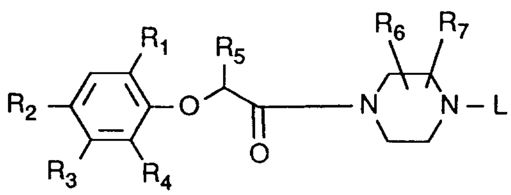

(X)

in welcher

$R^1$, $R^2$, $R^3$, $R^4$, $R^5$, $R^6$ und $R^7$ die in Anspruch 1 angegebene Bedeutung haben

und

L für eine Aminoschutzgruppe, vorzugsweise Benzyl steht,

nach Abspaltung der Aminoschutzgruppe, zunächst mit Verbindungen der allgemeinen Formel (VI) in inerten Lösemitteln, gegebenenfalls in Anwesenheit einer Base und/oder Hilfsstoffes umsetzt und anschließend wie in Anspruch 4 beschrieben einer Reduktion der Carbonylfunktion durchführt,

und im Fall der freien Carbonsäuren, den jeweiligen Ester mit Säuren oder Basen hydrolysiert oder verseift,

und im Fall einer Aminosäuregruppierung, diese nach denen in der Peptidchemie üblichen Methoden, beispielsweise durch Aktivierung der Carbonsäurefunktion, Blockierung und Deblockierung der entsprechenden Carbonsäure und/oder Aminosäurefunktionen und Kondensation, herstellt,

und im Fall der Substituenten $R^1$, $R^2$, $R^3$, $R^4$, $R^5$, $R^6$, $R^7$, $R^8$ und $R^9$, diese nach bekannten Verfahren, beispielsweise durch Reduktion, Alkylierung, Verseifung variiert.

**8.** Arzneimittel enthaltend eine oder mehrere der Verbindungen gemäß Anspruch 1.

**9.** Verwendung der Verbindung gemaß Anspruch 1 zur Herstellung von Arzneimitteln.